# EUROPEAN PATENT SPECIFICATION

(11) **EP 2 207 811 B1**
(45) Date of publication and mention of the grant of the patent: **04.01.2017**
(21) Application number: 08845286.7
(22) Date of filing: 03.11.2008
(51) Int. Cl.: C08B 37/10, A61K 31/727

(54) **NON-ANTICOAGULANT POLYSACCHARIDE COMPOSITIONS**
NICHT GERINNUNGSHEMMENDE POLYSACCHARIDZUSAMMENSETZUNGEN
COMPOSITIONS POLYSACCHARIDIQUES NON ANTICOAGULANTES

(30) Priority: 02.11.2007 US 985123 P
(43) Date of publication of application: 21.07.2010
(73) Proprietor: Momenta Pharmaceuticals, Inc., Cambridge, MA 02142 (US)
(72) Inventor: SUNDARAM, Mallik, Flemington NJ 07869 (US); KISHIMOTO, Takashi, Kei, Lexington MA 02420 (US); ROY, Sucharita, Tyngsboro MA 01879 (US)
(74) Representative: Simpson, Tobias Rutger
(86) International application number: PCT/US2008/082223
(87) International publication number: WO 2009/059283

(56) References cited:
- EP-A- 0 735 050
- WO-A-01/55221
- US-A1- 2006 172 968
- US-A1- 2008 051 567

## Description

### BACKGROUND

Heparin, a highly sulfated heparin-like glycosaminoglycan (HLGAG) produced by mast cells and isolated from natural sources, is a widely used clinical anticoagulant. However, the effects of natural, or unfractionated, heparin can be difficult to predict and patients must be monitored closely to prevent over- or under-anticoagulation. Low molecular weight heparins (LMWHs) obtained by various methods of fractionation or depolymerization of polymeric heparin have more predictable pharmacological action as anticoagulants, reduced side effects, sustained antithrombotic activity, and better bioavailability than unfractionated heparin (UFH). Several LMWHs are approved for outpatient treatment of thrombotic conditions.

There is increasing interest in the potential role of antithrombotic agents in the management of cancer patients. Results from several recent clinical trials have suggested a survival advantage for certain types of cancer patients treated with LMWHs (reviewed in Lemoine, 2005, Journal of Clinical Oncology, 23: 2119-20).

### SUMMARY OF THE INVENTION

The present invention provides polysaccharide preparations and related subject matter as defined in the appended claims.

More generally, the present disclosure is based, in part, on the development of polysaccharide preparations, e.g., preparations of polysaccharides derived from heparin, that lack substantial anticoagulant activity (e.g., preparations of polysaccharides that have substantially no anticoagulant activity) but retain activity in other non-coagulation mediated biological processes, and methods to produce them. These compounds can have one or more of the following features: 1) an anti-Xa activity and an anti-IIa activity each less than 50 IU/mg, and 2) anti-metastatic, anti-angiogenic, anti-fibrotic and/or anti-inflammatory activity. The polysaccharides disclosed herein can also have structural characteristics that distinguish them from other polysaccharides, (e.g., from commercially available heparins). For example, a polysaccharide preparation provided herein can have one or more of the following characteristics: the preparation has less than 50% glycol split uronic acid residues; the preparation has no more than 3 glycol split uronic acid residues (U_{G}) per polysaccharide chain; the preparation has greater than 40% U_{2S}H_{NS,6S} disaccharide residues; degree of desulfation of the preparation is less than 40%; one or more polysaccharide chains in the preparation have a 4,5-unsaturation of a non-reducing end uronic acid residue; one or more polysaccharide chains in the preparation have a 2,5-anhydromannitol residue at the reducing end; and the weight average molecular weight of the preparation is between 3,500 and 7,000 Da. This disclosure includes preparations having one or more of these properties and characteristics as well as methods of making and using such preparations. The disclosure also features methods of using such preparations.

Accordingly, in a first aspect of the disclosure, the disclosure features a polysaccharide preparation (e.g., a heparin-derived preparation) having the following characteristics: (a) a weight average chain molecular weight between 3,500 and 7,000 Da; (b) an anti-Xa activity and an anti-IIa activity each less than 50 IU/mg (e.g., an anti-Xa activity less than about 40 IU/mg, 30 IU/mg, 20 IU/mg, 15 IU/mg, or 10 IU/mg and an anti-IIa activity less than about 40 IU/mg, 30 IU/mg, 20 IU/mg, 10 IU/mg, 5 IU/mg, 4 IU/mg, or 3 IU/mg); and (c) less than 50% glycol split uronic acid residues (e.g., less than 40%, 30%, 25%, or 20% glycol split uronic acid residues) in the preparation. In some embodiments, the preparation contains between 5% and 50% glycol split uronic acid residues (e.g., between 5% and 40%, 5% and 30%, 10% and 50%, 10% and 40%, or 10% and 30% glycol split uronic acid residues).

In a second aspect, the disclosure features a polysaccharide preparation (e.g., a heparin-derived preparation) having the following characteristics: (a) a weight average chain molecular weight between 3,500 and 7,000 Da; (b) an anti-Xa activity and an anti-IIa activity each less than 50 IU/mg (e.g., an anti-Xa activity less than about 40 IU/mg, 30 IU/mg, 20 IU/mg, 15 IU/mg, or 10 IU/mg and an anti-IIa activity less than about 40 IU/mg, 30 IU/mg, 20 IU/mg, 10 IU/mg, 5 IU/mg, 4 IU/mg, or 3 IU/mg); and (c) the polysaccharide chains of the preparation have no more than 3 glycol split uronic acid residues (U_{G}) per polysaccharide chain (e.g., each polysaccharide chain has no more than 2 or no more than 1 glycol split uronic acid residue (U_{G}) per polysaccharide chain).

In a third aspect, the disclosure features a polysaccharide preparation (e.g., a heparin-derived preparation) having the following characteristics: (a) a weight average chain molecular weight between 3,500 and 7,000 Da; (b) an anti-Xa activity and an anti-IIa activity each less than 50 IU/mg (e.g., an anti-Xa activity less than about 40 IU/mg, 30 IU/mg, 20 IU/mg, 15 IU/mg, or 10 IU/mg and an anti-IIa activity less than about 40 IU/mg, 30 IU/mg, 20 IU/mg, 10 IU/mg, 5 IU/mg, 4 IU/mg, or 3 IU/mg); and (c) polysaccharide chains of the preparation have on average no more than 3 glycol split uronic acid residues (U_{G}) per polysaccharide chain (e.g., on average no more than 2.5, no more than 2, no more than 1.5, or no more than 1 glycol split uronic acid residues (U_{G}) per polysaccharide chain.

In a fourth aspect, the disclosure features a polysaccharide preparation (e.g., a heparin-derived preparation) having the following characteristics: (a) a weight average chain molecular weight between 3,500 and 7,000 Da; (b) an anti-Xa activity and an anti-IIa activity each less than 50 IU/mg (e.g., an anti-Xa activity less than about 40 IU/mg, 30 IU/mg, 20 IU/mg, 15 IU/mg, or 10 IU/mg and an anti-IIa activity less than about 40 IU/mg, 30 IU/mg, 20 IU/mg, 10 IU/mg, 5 IU/mg, 4 IU/mg, or 3 IU/mg); and (c) the preparation has greater than 40% U_{2S}H_{NS,6S} disaccharide residues (e.g., greater than 50%, 60%, 70%, or 80% U_{2S}H_{NS,6S} disaccharide residues). In some embodiments, the preparation has a degree of desulfation less than 40% (e.g., less than 30%, 20%, or 10%).

In a fifth aspect, the disclosure features a polysaccharide preparation (e.g., a heparin-derived preparation) lacking substantial anticoagulant activity (e.g., having substantially no anticoagulant activity), wherein the preparation includes polysaccharides that include Formula I:

[U_{w}-H_{x,y,z}]ₘ~[U_{G}-H_{x,y,z}]n

wherein U indicates a uronic acid residue and H indicates a hexosamine residue;
m and n are integers such that
m = 4-16 (e.g., 4-8, 4-9, 4-10, 4-11, 4-12, 4-13, 4-14, or 4-15), and
n = 1-4 (e.g., 1-2 or 1-3);
w = -2OS or -2OH;
x = NS or NAc;
y = -3OS or -3OH;
z = -6OS or -6OH; and 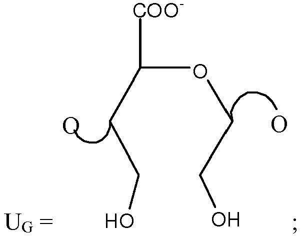 wherein the symbol ~ indicates that the units marked m and n are distributed along the polysaccharide chain and are not necessarily in sequence, wherein w, x, y, and z are each the same or different on each unit marked m, and wherein x, y, and z are each the same or different on each unit marked n.

In a sixth aspect, the disclosure features a polysaccharide preparation (e.g., a heparin-derived preparation) lacking substantial anticoagulant activity (e.g., having substantially noanticoagulant activity) and having antimetastatic activity, wherein the preparation includes polysaccharides that include Formula II:

[U_{w}-H_{x,y,z}]ₘ-[U_{G}-H_{x,y,z}]ₙ-[U_{w}H_{x,y,z}]ₒ-[U_{G}-H_{x,y,z}]ₚ-[U_{w}-H_{x,y,z}]_{q}

wherein U indicates a uronic acid residue and H indicates a hexosamine residue;
wherein m-r are integers such that:
   m = 0-10;
   n= 0-3;
   o = 0-10;
   p = 0-3;
   q = 0-10;
   w = -2OS or -2OH;
   x = NS or NAc;
   y = -3OS or -3OH;
   z = -6OS or -6OH;
   and 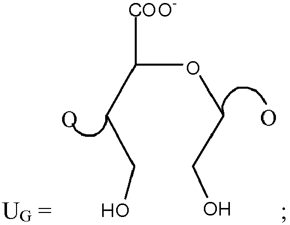 wherein w, x, y, and z are each the same or different on each unit marked m, n, o, p, or q. In some embodiments, the sum of n + p is less than or equal to 4 (e.g., less than or equal to 3, 2, 1, or 0). In some embodiments, the preparation has a weight average chain molecular weight between 3,500 and 7,000 Da.

The disclosure also includes pharmaceutically acceptable salts of any of the preparations described herein (e.g., described above) and compositions (e.g., pharmaceutical compositions) that comprise the preparations described herein and/or their pharmaceutically acceptable salts.

Any of the preparations described herein, e.g., described above, can have other properties. E.g., one of the above described preparations or pharmaceutical compositions can further have one or more of the functional or structural properties set out below.

In one embodiment, at least one of the polysaccharide chains in the preparation has one of the following structures at the non-reducing end: 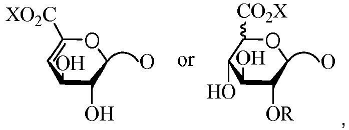 wherein X is H or Me and R is H or SO₃. For example, about 10%, 20%, 30%, 40%, 50%, 60%, 70%, 80%, 90%, or substantially all of the non-reducing ends of the preparation or pharmaceutical composition have the structure.

In one embodiment, at least one of the polysaccharide chains in the preparation or pharmaceutical composition includes a 2,5-anhydromannitol residue at the reducing end. For example, about 10%, 20%, 30%, 40%, 50%, 60%, 70%, 80%, 90%, or substantially all of the polysaccharide chains in the preparation or pharmaceutical composition include a 2,5-anhydromannitol residue at the reducing end.

In one embodiment, the preparation or pharmaceutical composition has a molecular weight distribution such that 10-50% (e.g., 10-40%, 10-30%, 15-30% or 15-25%) of the oligosaccharides of the preparation have a molecular weight < 3000 Da; 40-65% (e.g., 45-65%, 50-65%, or 55-65%) of the oligosaccharides have a molecular weight between 3000-8000 Da, and 5-30% (e.g., 10-30%, 15-30%, 10-25%, or 15-25%) of the oligosaccharides have a molecular weight > 8000 Da.

In one embodiment, the preparation has a polydispersity of about 1.2 to 1.7 (e.g., about 1.3 to 1.7, 1.2 to 1.6, or 1.3 to 1.6).

In one embodiment, the preparation or composition has anti-metastatic activity.

In one embodiment, the preparation or composition binds specifically to or inhibits an activity of one or more of: VEGF, FGF, SDF-1-α, or P-selectin.

In one embodiment, the preparation or composition has a sodium content less than 30%, 25%, 20%, 15%, 10%. In one embodiment, the preparation or composition comprises: less than 20 ppm, 15 ppm, 10 ppm, 5 ppm iodine; less than 30%, 25%, 20%, 15%, 10% sulfur; less than 50, 40, 30, 20, 15 ppm boron.

In another aspect, the disclosure features methods of making a preparation. The methods include: combining UFH and nitrous acid (HONO) to produce a polysaccharide preparation; and, following nitrous acid treatment, performing reactions to produce a glycol split of at least a portion of the uronic acid residues in the preparation.

In another aspect, methods of making a preparation include: depolymerizing an UFH (e.g., by chemical hydrolysis or enzymatic depolymerization); and, following depolymerization, performing reactions to produce a glycol split of at least a portion of the uronic acid residues in the preparation.

In one embodiment, reactions to produce a glycol split of at least a portion of the uronic residues in the preparation include oxidizing the polysaccharide preparation with periodate; and reducing the oxidized polysaccharide preparation with sodium borohydride. For example, the methods include oxidizing the polysaccharide preparation with periodate for about 10-20 hours at a temperature of about 0-10 °C; and following oxidation, reducing the sample with sodium borohydride for about 1 hour at a pH of about 5.0-8.0 at a temperature of about 0-10 °C.

In another aspect, the disclosure features methods of manufacturing a preparation. The methods include: (1) depolymerizing an unfractionated heparin (UFH) (e.g., by nitrous acid depolymerization, hydrolytic depolymerization, or enzymatic depolymerization) to yield a polysaccharide preparation; (2) oxidizing the polysaccharide preparation with periodate; (3) reducing the oxidized polysaccharide preparation with sodium borohydride; and (4) isolating the polysaccharide preparation (e.g., by precipitating with a salt and a polar organic solvent, or by subjecting to a chromatographic separation or purification), to thereby make a preparation.

In one embodiment, the step of depolymerizing includes treating the UFH with about 0.01 to 0.05 M (e.g., about 0.02 to 0.04 M) nitrous acid at a pH of about 2 to 4 for about 1 to 5 hours at a temperature of about 10 to 30 °C .

In one embodiment, the step of oxidizing includes treating the polysaccharide preparation with about 0.05 to 0.2 M periodate for about 10 to 20 hours at a temperature of about 0 to 10 °C.

In one embodiment, the step of reducing comprises treating the oxidized polysaccharide preparation with about 0.5 to 2.0% (w/v) sodium borohydride for about 0.5 to 3 hours at a pH of about 6.0 to 7.0 and a temperature of about 0 to 10 °C.

In one embodiment, a method of making or manufacturing a polysaccharide preparation includes reducing the amount of boron in the preparation.

In one embodiment, the preparation is evaluated for a biological activity, e.g., anti-metastatic activity; binding to any ofVEGF, FGF, SDF-1α, and P-selectin; or inhibition of an activity of any ofVEGF, FGF, SDF-1α, and P-selectin.

A polysaccharide preparation that lacks substantial anticoagulant activity, as used herein, is one that has anti-Xa and anti-IIa activity each less than 100 IU/mg (e.g., less than 80 IU/mg, 70 IU/mg, or 60 IU/mg). In some embodiments, the polysaccharide preparation has substantially no anticoagulant activity, i.e., anti-Xa and anti-IIa activity each less than 50 IU/mg. In some embodiments, the polysaccharide preparation has anti-Xa and anti-IIa activity each less than 40, 30, 20, 25, 20, 15, 10 or 5 IU/mg.

The degree of desulfation, as used herein, is defined as the percent reduction in moles of sulfate per moles of disaccharide unit as compared to unfractionated heparin.

The degree of sulfation, as used herein, is defined as the average number of moles of sulfate per moles of disaccharide unit.

In another aspect, the disclosure features a polysaccharide preparation made by a method described herein.

In another aspect, the disclosure includes an intermediate or reaction mixture from any of the methods for making or analyzing a polysaccharide preparation described herein.

In another aspect, the disclosure features a pharmaceutical composition that includes a polysaccharide preparation described herein.

In one embodiment, the pharmaceutical composition further includes a pharmaceutically acceptable carrier.

In another aspect, the disclosure features a method of treating a subject that includes administering a therapeutically effective amount of a polysaccharide preparation disclosed herein to the subject. The terms "treating", "treatment", and the like, mean administering the preparation to a subject or a cell or tissue of a subject in order to obtain a desired pharmacological, physiological or clinical effect. Treatment with a polysaccharide preparation described herein may lessen, reduce, mitigate, ameliorate, delay, or prevent an existing unwanted condition or the onset or a symptom thereof. A "therapeutically effective amount" refers to an amount effective, at dosages and for periods of time necessary, to achieve the desired pharmacological, physiological or clinical effect in the subject.

The disclosure includes methods for treating a subject having, or at risk of having, a metastatic disorder (e.g., a cancer, e.g., a carcinoma or other solid and hematological cancer). In those subjects, treatment may include, but is not limited to, inhibited tumor growth, reduction in tumor mass, reduction in size or number of metastatic lesions, inhibited development of new metastatic lesions, prolonged survival, prolonged progression-free survival, prolonged time to progression, and/or enhanced quality of life. In another embodiment, the subject may have a disorder or condition selected from the group consisting of: an inflammatory disorder, an autoimmune disease, a fibrotic or fibroproliferative disorder or an atopic disorder. Examples of inflammatory disorders include but are not limited to chronic obstructive pulmonary disease, asthma, rheumatoid arthritis, inflammatory bowel disease (including Crohns disease and ulcerative colitis), multiple sclerosis, psoriasis, ischemia-reperfusion injuries, septic shock, age-related macular degeneration, atherosclerosis, Alzheimer's disease, cardiovascular disease, vasculitis, type I and II diabetes, metabolic syndrome, diabetic retinopathy, restenosis. Examples of autoimmune diseases include but are not limited to asthma, rheumatoid arthritis, inflammatory bowel disease, multiple sclerosis, psoriasis, type I diabetes, systemic lupus erythematosus (SLE), Sjögren's syndrome, Hashimoto's thyroiditis, Graves' disease, Guillain-Barré syndrome, autoimmune hepatitis, Myasthenia gravis. Examples of fibrotic diseases include but are not limited to scleroderma, chronic obstructive pulmonary disease, diabetic nephropathy, sarcoidosis, idiopathic pulmonary fibrosis, cirrhosis, cystic fibrosis, neurofibromatosis, endometriosis, post-operative fibroids, restenosis. Examples of atopic disease include but are not limited to atopic dermatitis, atopic asthma, and allergic rhinitis. The compositions of the disclosure are administered to a subject having or at risk of developing one or more of the diseases in an effective amount for treating the disorder or condition.

In a preferred embodiment, the subject has, or is at risk of having, a cancer or metastatic disorder (e.g., a carcinoma). For example, the subject has a primary tumor and has, or is at risk of having, a metastasis of that primary tumor.

In one embodiment, the polysaccharide preparation is administered intravenously or subcutaneously or is inhaled.

In one embodiment, the polysaccharide preparation is administered in combination with another therapy, e.g., another therapeutic agent, e.g., a cytotoxic or cytostatic agent, and combinations thereof.

In one embodiment, the polysaccharide preparation is administered chronically, e.g., at least twice over a specific period of time, e.g., at least twice during a period of six months. In one embodiment, a polysaccharide preparation is administered twice over a period of one week, two weeks, three weeks, one month, two months, three months, six months, one year, or even longer. The polysaccharide preparation can be administered daily (e.g., once, twice, or three or four times daily), once every other day, weekly (e.g., once, twice, or three times a week), once every other week, monthly, or any other chronic administration schedule.

For any of the ranges described herein, e.g., for a given structure or activity, the ranges can be those ranges disclosed as well as other ranges. For example, a range constructed from a lower endpoint of one range, e.g., for a given building block or activity, can be combined with the upper endpoint of another range, e.g., for the given building block or activity, to give a range.

An "isolated" or "purified" polysaccharide preparation is substantially free of cellular material or other contaminating proteins from the cell or tissue source from which the polysaccharide is derived, or substantially free from chemical precursors or other chemicals when chemically synthesized. "Substantially free" means that a preparation is at least 50% pure (wt/wt). In a preferred embodiment, the preparation has less than about 30%, 20%, 10% and more preferably 5% (by dry weight), of non-heparin-derived polysaccharides, proteins or chemical precursors or other chemicals, e.g., from manufacture. These are also referred to herein as "contaminants." Examples of contaminants that can be present in a polysaccharide preparation provided herein include, but are not limited to, sodium, sulfur, boron, enzyme (e.g., a heparinase enzyme), methanol, ethanol, iodine, and chloride.

Other features and advantages of the invention will be apparent from the following detailed description, and from the claims.

### BRIEF DESCRIPTION OF THE DRAWINGS

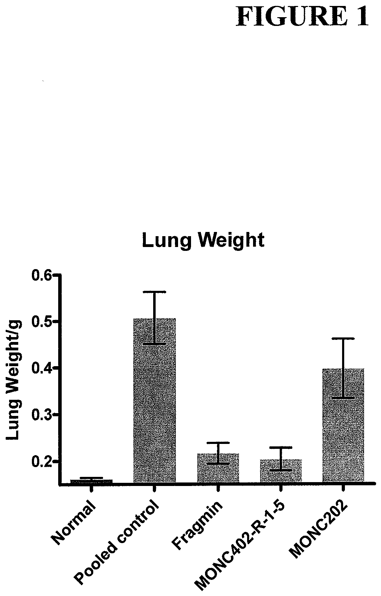

Figure 1 is a bar graph showing the effect of a polysaccharide preparation described herein in a murine melanoma experimental metastasis (B16F10 i.v.) model. Lung tumor burden (lung weight - normal lung weight) was determined for female C57BL/6 mice (9-10 weeks old) challenged with i.v. injection of 2x10⁵ B16F10 cells and pretreated with a single dose (10 mg/kg) of MONC402 (batch R-1-5), dalteparin (Fragmin®), or MONC 202 (negative control, N-desulfated polysaccharide) immediately before injection. "Normal" designates unchallenged and untreated mice.

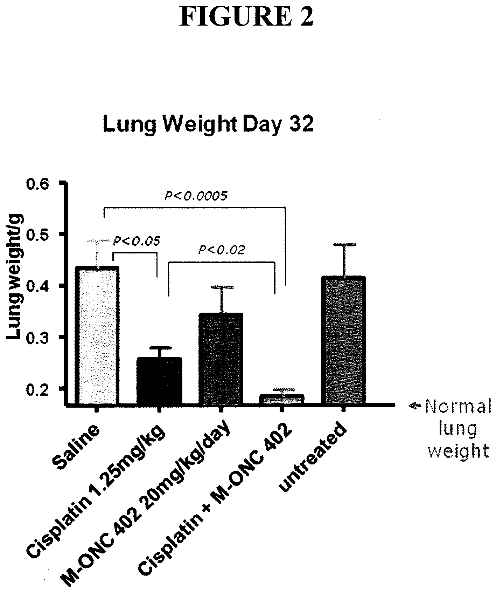

Figure 2 is a bar graph showing the effect of a polysaccharide preparation described herein in a 4T1 therapeutic model of breast cancer metastasis to the lung. Lung tumor burden (lung weight - normal lung weight) was determined on day 32 for female BALB/c mice (8 weeks old) challenged with intra-mammary fat pad injection of 8x10⁴ 4T1 cells and treated as indicated starting on day 4.

### DETAILED DESCRIPTION

### Optimized Polysaccharides

In many clinical settings, commercially available LMWH preparations are preferred over UFH preparations as anticoagulants because LMWHs have more predictable pharmacokinetics and can be administered subcutaneously. However, because of the potential for bleeding complications due to their anticoagulant effects, currently available LMWH preparations are less suitable for therapy of non-coagulation mediated disorders, and/or for disorders that may require higher doses or chronic dosing regimens. The disclosure features polysaccharide preparations designed to lack substantial anticoagulant activity while retaining clinically advantageous properties. Properties of the polysaccharide preparations include, e.g., lacking substantial anticoagulant activity, e.g., having substantially no anticoagulant activity (e.g., anti-IIa activity less than 50 IU/mg, anti-Xa activity less than 50 IU/mg), and having anti-metastatic, anti-angiogenic and/or anti-inflammatory activity.

Examples of such polysaccharide preparations include chains that include the following:

[U_{w}-H_{x,y,z}]ₘ~[U_{G}-H_{x,y,z}]ₙ

wherein U indicates a uronic acid residue and H indicates a hexosamine residue, wherein m and n are integers such that m = 6-18, and n = 1-4, w = -2OS or -2OH, x = -NS or NAc, y = -3OS or -3OH, z = -6OS or -6OH, and 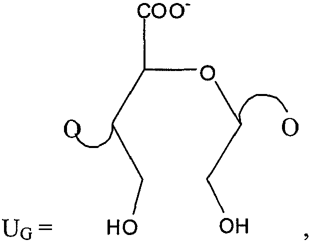 wherein the symbol ~ indicates that the units marked m and n are distributed along the polysaccharide chain and are not necessarily in sequence, wherein w, x, y, and z are each the same or different on each unit marked m, and wherein x, y, and z are each the same or different on each unit marked n; and

[U_{w}-Hx_{,y,z}]ₘ-[U_{G}-H_{x,y,z}]ₙ-[U_{w-}H_{x,y,z}]ₒ-[U_{G}-H_{x,y,z}]ₚ-[U_{w}-H_{x,y,z}]_{q}

wherein U indicates a uronic acid residue and H indicates a hexosamine residue, wherein m-r are integers such that: m = 0-10, n= 0- 3, o = 0-10, p = 0-3, q = 0-10, w = -2OS or -2OH, x = -NS or -NAc, y = -3OS or -3OH, z = -6OS or -6OH, and 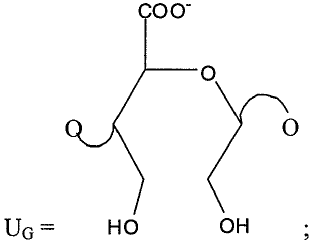 wherein w, x, y, and z are each the same or different on each unit marked m, n, o, p, or q.

### Anti-IIa Activity

Polysaccharide preparations are disclosed herein that provide substantially reduced anti-IIa activity, e.g., anti-IIa activity of about 0 to 50 IU/mg, about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, about 5 to 20 IU/mg. Anti-IIa activity is calculated in International Units of anti- IIa activity per milligram using statistical methods for parallel line assays. The anti-IIa activity levels described herein are measured using the following principle.

Polysaccharide (PS) + ATIII → [PS • ATIII] IIa

PS • ATIII→[PS • ATIII • IIa] + IIa (Excess)
IIa (Excess) + Substrate → Peptide + pNA (measured spectrophotometrically)

Anti-factor IIa activity is determined by the sample potentiating effect on antithrombin (ATIII) in the inhibition of thrombin. Thrombin excess can be indirectly spectrophotometrically measured. The anti-factor IIa activity can be measured, e.g., on a Diagnostica Stago analyzer or on an ACL Futura3 Coagulation system, with reagents from Chromogenix (S-2238 substrate, Thrombin (53 nkat/vial), and Antithrombin), or on any equivalent system. Analyzer response is calibrated using the 2nd International Standard for Low Molecular Weight Heparin.

### Anti-Xa Activity

Preferably, a polysaccharide preparation provided herein has an anti-Xa activity of about 0 to 50 IU/mg, e.g., about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, about 5 to 20 IU/mg. Anti-Xa activity of a preparation is calculated in International Units of anti-factor Xa activity per milligram using statistical methods for parallel line assays. The anti-factor Xa activity of preparations described herein is measured using the following principle:

PS + ATIII → [PS • ATIII] FXa

PS • ATIII → [PS • ATIII • FXa] + FXa(Excess)
FXa (Excess) + Substrate → Peptide + pNA (measured spectrophotometrically)

The anti-factor Xa activity is determined by the sample potentiating effect on antithrombin (ATIII) in the inhibition of activated Factor Xa (FXa). Factor Xa excess can be indirectly spectrophotometrically measured. Anti-factor Xa activity can be measured, e.g., on a Diagnostica Stago analyzer with the Stachrom® Heparin Test kit, on an ACL Futura3 Coagulation system with the Coatest® Heparin Kit from Chromogenix, or on any equivalent system. Analyzer response can be calibrated using the NIBSC International Standard for Low Molecular Weight Heparin.

### Molecular Weight and Chain Length

When weight average molecular weight of a preparation is determined, a weight average molecular weight of about 3500 to 7000 Da, about 3500 to 6300 Da, preferably about 4000 to 6000 Da, about 4200 to 5900, or about 4300 to 5800 Da, indicates that a significant number of chains in the polysaccharide preparation are of sufficient chain length.

"Weight average molecular weight" as used herein refers to the weight average in daltons of chains of uronic acid/hexosamine disaccharide repeats. The presence of non-uronic acid and/or non-hexosamine building blocks are not included in determining the weight average molecular weight. Thus, the molecular weight of non-uronic acid and non-hexosamine building blocks within a chain or chains in the preparation should not be included in determining the weight average molecular weight. The weight average molecular weight (M_{w}) is calculated from the following equation: M_{w} = ∑(cᵢmᵢ)/∑cᵢ. The variable cᵢ is the concentration of the polymer in slice *i* and mᵢ is the molecular weight of the polymer in slice *i.* The summations are taken over a chromatographic peak, which contains many slices of data. A slice of data can be pictured as a vertical line on a plot of chromatographic peak versus time. The elution peak can therefore be divided into many slices. The weight average molecular weight calculation is average dependant on the summation of all slices of the concentration and molecular weight. The weight average molar weight can be measured, e.g., using the Wyatt Astra software or any appropriate software. The weight average molecular weights described herein are determined by high liquid chromatography with two columns in series, for example a TSK G3000 SWXL and a G2000 SWXL, coupled with a multi angle light scattering (MALS) detector and a refractometric detector in series. The eluent used is a 0.2 M sodium sulfate, pH 5.0, and a flow rate of 0.5 mL/min.

A determination of whether a polysaccharide preparation includes chains of sufficient chain length can be made, for example, by determining the average chain length of the chains in the preparation and/or by determining the weight average molecular weight of chains within the preparation. When average chain length is determined, an average chain length of about 5 to 22, e.g., about 7 to 18, typically about 7 to 14 or 8 to 13 disaccharide repeats, indicates that a significant number of chains in the preparation are of sufficient chain length.

"Average chain length" as used herein refers to the average chain length of uronic acid/hexosamine disaccharide repeats that occur within a chain. The presence of non-uronic acid and/or non-hexosamine building blocks (e.g., attached PEG moieties) are not included in determining the average chain length. Average chain length is determined by dividing the number average molecular weight (Mn) by the number average molecular weight for a disaccharide (500 Da). Methods of determining number average molecular weight are described below using SEC MALS.

### Glycol Split Uronic Acids

A polysaccharide preparation described herein can include an opening of the glycoside ring, conventionally called reduction-oxidation (RO) derivatives. In these preparations, one or more glycoside rings having vicinyl diols that are opened, e.g., at the bond between C2 and C3, by means of an oxidation action, followed by a reduction. The compounds referred to herein will also be called "Glycol Split" derivatives.

In a further embodiment, the glycol split residues lend themselves to the subsequent functionalization. Therefore, the compounds may also bear equal or different groups, in place of the primary hydroxy groups deriving from glycol split, for
example, aldehyde groups, methoxy groups, or oligosaccharide or peptide groups, ranging from a single saccharide or amino acid to more than one unit of length, e.g., 2 or 3 units.

In some embodiments, fewer than 50% of the uronic acid residues are glycol split uronic acid residues (e.g., less than 40%, 30%, 25%, or 20% of the uronic acid residues are glycol split uronic acid residues).

### Reducing End Structures

In some instances, at least about 50% of the chains in a polysaccharide preparation described herein have a modified reducing end structure such as a 2,5-anhydromannose residue or a 2,5-anhydromannose that has been reduced to form an alcohol. In some embodiments, at least about 55%, 60%, 65%, 70%, 75%, 80%, 85%, 90%, or 95% of the chains in the preparation have a modified reducing end structure, such that the reducing end includes a 2,5-anhydromannose residue or a 2,5-anhydromannose that has been reduced to form an alcohol.

### Polydispersity

The polydispersity of polysaccharide preparations provided herein is about 2 or less, e.g., 1.7 or less, e.g., about 1.7 or 1.6 to 1.2, about 1.4-1.5, and numbers in between.

The term "polydisperse" or "polydispersity" refers to the weight average molecular weight of a composition (Mw) divided by the number average molecular weight (Mn). The number average molecular weight (Mn) is calculated from the following equation: Mn = ∑ci/(∑ci/mi). The variable ci is the concentration of the polysaccharide in slice i and Mi is the molecular weight of the polysaccharide in slice i. The summations are taken over a chromatographic peak, which contains many slices of data. A slice of data can be pictured as a vertical line on a plot of chromatographic peak versus time. The elution peak can therefore be divided into many slices. The number average molecular weight is a calculation dependent on the molecular weight and concentration at each slice of data. Methods of determining weight average molecular weight are described above, and were used to determine polydispersity as well.

### Methods of Making Polysaccharide Preparations

Various methods of making polysaccharide preparations, e.g., a preparation described herein, are also contemplated. One method includes providing a precursor heparin preparation having a weight average molecular weight of greater than 7000 Da or a chain length of greater than 7 to 18 disaccharides, and processing the precursor heparin preparation (e.g., by enzymatic or chemical depolymerization, e.g., by nitrous acid depolymerization) to obtain a polysaccharide preparation having a weight average molecular weight of about 3000 to 7000 Da or an average chain length of about 7 to 18 disaccharides. For example, the precursor heparin preparation can be unfractionated heparin.

The precursor heparin preparation can be processed by a method comprising depolymerization (e.g., by nitrous acid treatment, hydrolysis, or enzymatic depolymerization) followed by a glycol split reaction. Nitrous acid depolymerization can be accomplished, e.g., by treating the precursor heparin preparation (e.g., UFH) with nitrous acid (e.g., about 0.02 to 0.04 M nitrous acid) at a pH of about 2 to 4 for a specified period of time (e.g., about 1 to 5 hours) at a temperature of about 10 to 30 °C. The glycol split reaction involves periodate oxidation using periodate (e.g., about 0.05 M to 0.2 M sodium periodate) for about 10 to 20 hours at a temperature of about 0 to 10 °C. In some embodiments, residual impurities such as salts or diethylene glycol (DEG) can be subsequently removed by a chromatographic method, e.g. gel filtration chromatography. Optionally, the oxidized preparation is then reduced by treatment with a reducing agent (e.g., about 0.5 to 2.0% (w/v) sodium borohydride) for about 0.5 to 3 hours at a pH of about 6.0 to 7.0 and a temperature of about 0 to 10 °C.

A precursor heparin preparation can be processed using enzymatic digestion, chemical digestion or combinations thereof. Examples of chemical digestion include oxidative depolymerization, e.g., with H₂O₂ or Cu⁺ and H₂O₂, deaminative cleavage, e.g., with isoamyl nitrite or nitrous acid, β-eliminative cleavage, e.g., with benzyl ester, and/or by alkaline treatment. Enzymatic digestion can include the use of one or more heparin degrading enzymes. For example, the heparin degrading enzyme(s) can be, e.g., one or more heparinase, heparin lyase, heparin sulfate glycoaminoglycan (HSGAG) lyase, a lyase described as a glycoaminoglycan (GAG) lyase that can also degrade heparin. Preferably, the enzyme cleaves at one or more glycosidic linkages of unsulfated uronic acids.

### Biological Activities

The preparations described herein have anti-metastatic activity as assayed in an animal model of metastasis in which B16F10 melanoma cells injected into the tail veins of C57/BL mice arrest in the lungs and proliferate as discrete pulmonary foci. This assay is generally described in Gabri et al., 2006, Clin. Cancer Res., 12:7092-98. A preparation may additionally have activity in other experimental models of metastasis, including the C170HM2 assay, in which C170HM2 human colorectal cancer line cells are injected into the peritoneal cavity, where the primary site of metastasis is to the liver. The preparations described herein may also show anti-metastatic activity in spontaneous models of metastasis, such as the AP5LV model, in which AP5LV human colorectal cancer cells are implanted into the peritoneal wall and exhibit spontaneous metastasis to the lung, or the 4T1 model, in which 4T1 murine mammary carcinoma cells implanted in to the mammary fat pad exhibit spontaneous metastasis to the lung and other organs.

The preparations described herein can bind to and/or modulate (e.g., inhibit) an activity of one or more of VEGF, FGF, SDF-1α, and P-selectin. In some embodiments, interaction of the preparation with (e.g., binding to) a target protein (e.g., VEGF, FGF, SDF-1α, or P-selectin) can be assayed, e.g., in vitro, e.g., using methods known in the art. Numerous methods and techniques to detect binding or modulation (e.g., inhibition) of activity are known, e.g., standard receptor competition assays, fluorescence energy transfer (FET), fluorescence resonance energy transfer (FRET) (see, for example, U.S. Pat. No. 5,631,169; U.S. Pat. No. 4,868,103), and fluorescence polarization (FP). In some embodiments, evaluating binding of a polysaccharide preparation to a target protein can include a real-time monitoring of the binding interaction, e.g., using Biomolecular Interaction Analysis (BIA) (see, e.g., Sjolander and Urbaniczky (1991) Anal. Chem., 63:2338-2345 and Szabo et al. (1995) Curr. Opin. Struct. Biol., 5:699-705). Surface plasmon resonance or "BIA" detects biospecific interactions in real time, without labeling any of the interactants (e.g., BIAcore).

Activities of VEGF, FGF, and P-selectin on cells in vitro and in vivo are well known in the art. The ability of a polysaccharide preparation to modulate (e.g., inhibit) an activity of VEGF, FGF, or P-selectin can be assayed in vitro or in a cell-based assay or in vivo in an organism. For example, the ability of a polysaccharide preparation to modulate (e.g., inhibit) the activity of VEGF, FGF, or P-selectin to modulate (e.g., stimulate) the proliferation of endothelial cells, e.g., human umbilical vein epithelial cells, can be assayed. Exemplary methods of determining modulation of FGF activity can be found in U.S. Patent No. 5,733,893. A cell-based assay can be performed using a single cell, or a collection of at least two or more cells. The cell can be a yeast cell (e.g., *Saccharomyces cerevisiae*) or a mammalian cell, e.g., a cell line.

### Pharmaceutical Compositions

Compositions, e.g., pharmaceutically acceptable compositions, which include a preparation described herein, formulated together with a pharmaceutically acceptable carrier, are provided.

As used herein, "pharmaceutically acceptable carrier" includes any and all solvents, dispersion media, isotonic and absorption delaying agents, and the like that are physiologically compatible with parenteral administration. The carrier can be suitable for any parenteral administration, e.g., intravenous, intramuscular, subcutaneous, intraocular, rectal, inhaled or spinal administration (e.g., by injection or infusion).

The compositions of this disclosure may be in a variety of forms. These include, for example, liquid, semi-solid and solid dosage forms, such as liquid solutions (e.g., injectable and infusible solutions), dispersions or suspensions, and liposomes. The preferred form depends on the intended mode of administration and therapeutic application. Typical preferred compositions are in the form of injectable or infusible solutions. The preferred mode of administration is parenteral (e.g., intravenous, subcutaneous, intraocular, intraperitoneal, intramuscular). In a preferred embodiment, the preparation is administered by intravenous infusion or injection. In another preferred embodiment, the preparation is administered by intramuscular or subcutaneous injection.

The phrases "parenteral administration" and "administered parenterally" as used herein means modes of administration other than enteral and topical administration, usually by injection, and includes, without limitation, intravenous, intramuscular, subcutaneous, intraarterial, intrathecal, intracapsular, intraorbital, intravitreous, intracardiac, intradermal, intraperitoneal, transtracheal, inhaled, subcutaneous, subcuticular, intraarticular, subcapsular, subarachnoid, intraspinal, epidural and intrasternal injection and infusion.

Therapeutic compositions typically should be sterile and stable under the conditions of manufacture and storage. The composition can be formulated as a solution, microemulsion, dispersion, liposome, or other ordered structure suitable to high concentration. Sterile injectable solutions can be prepared by incorporating the active compound (i.e., polysaccharide preparation) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, various polymers, monostearate salts and gelatin.

For many therapeutic applications, the preferred route/mode of administration is intravenous injection or infusion. As will be appreciated by the skilled artisan, the route and/or mode of administration will vary depending upon the desired results.

Formulations for injection may be presented in unit dosage form, e.g., in ampoules, syringes, syringe pens, or in multi-dose containers, e.g., with an added preservative. The compositions may take such forms as suspensions, solutions or emulsions in oily or aqueous vehicles, and may contain formulatory agents such as suspending, stabilizing and/or dispersing agents.

For administration by inhalation, the preparation may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch. In addition, dry powder formations for inhalation therapy are within the scope of the disclosure. Such dry powder formulations may be prepared as disclosed, e.g., in WO 02/32406.

In addition to the compositions described previously, the compounds may also be formulated as a depot preparation. Such long-acting formulations may be formulated with suitable polymeric or hydrophobic materials (for example, as an emulsion in an acceptable oil) or ion exchange resins, or as sparingly soluble derivatives, for example, as a sparingly soluble salt.

The pharmaceutical compositions also may comprise suitable solid or gel phase carriers or excipients. The compositions can be included in a container, pack, or dispenser together with instructions for administration.

The preparation can also be administered with short or long term implantation devices, e.g., a stent. The preparation can be implanted subcutaneously, can be implanted into tissues or organs (e.g., the coronary artery, carotid artery, renal artery and other peripheral arteries, veins, kidney, heart cornea, vitreous, cerebrum, etc.), or can be implanted in physiological spaces around tissues and organs (e.g., kidney capsule, pericardium, thoracic or peritoneal space).

The preparation can also be used to coat various medical devices. For example, the preparation can be used to coat a stent or extracorporeal circuit. Such formulations of the preparations may include using, e.g., controlled release beads, gel or microspheres as well as various polymers such as PLGA, cellulose, alginate or other polysaccharides.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions.

The pharmaceutical compositions of the disclosure may include a therapeutically effective amount of a preparation. A therapeutically effective amount of the preparation may vary according to factors such as the disease state, age, sex, and weight of the individual and can include more than one unit dose. A therapeutically effective amount is also one in which any toxic or detrimental effects of the preparation are outweighed by the therapeutically beneficial effects. A therapeutically effective amount may inhibit a measurable parameter, e.g., VEGF activity, FGF activity, P-selectin activity, or size or rate of growth of metastatic lesions, e.g., by at least about 20%, more preferably by at least about 25%, 30%, 40%, even more preferably by at least about 50%, 60%, and still more preferably by at least about 70%, 80% relative to untreated subjects. The ability of a compound to inhibit a measurable parameter, e.g., metastasis or angiogenesis, can be evaluated in an animal model system or in a human (e.g., in a pre-clinical model or a clinical trial). Alternatively, a property of a composition can be evaluated by examining the activity of the compound in an *in vitro* assay. Exemplary doses for intravenous or subcutaneous administration of the polysaccharide preparation are about 0.03 mg/kg to 0.45 mg/kg, e.g., 0.03 mg/kg, 0.05 mg/kg, 0.1 mg/kg, 0.15 mg/kg, 0.2 mg/kg, 0.22 mg/kg, 0.25 mg/kg, 0.27 mg/kg, 0.3 mg/kg, 0.35 mg/kg, 0.37 mg/kg, 0.4 mg/kg, 0.44 mg/kg, preferably about 0.1 mg/kg, 0.15 mg/kg, 0.2 mg/kg, 0.25 mg/kg, 0.3mg/kg, 0.35 mg/kg, 0.4 mg/kg, 0.44 mg/kg, 0.47 mg/kg, 0.5 mg/kg, 0.55 mg/kg, 0.60 mg/kg, 0.7 mg/kg, preferably about 0.30 to 0.50 mg/kg, e.g., 0.30mg/kg, 0.35mg/kg, 0.40 mg/kg, 0.42 mg/kg, 0.44 mg/kg, 0.47 mg/kg or 0.50 mg/kg. In some embodiments, the polysaccharide preparation can be administered at a dose between 1-80 mg/kg, between 1-40 mg/kg, between 1-30 mg/kg, e.g., between 5-50 mg/kg/day.

Also within the scope of the disclosure are kits comprising a polysaccharide preparation provided herein. The kit can include one or more other elements including: instructions for use; other reagents, e.g., a therapeutic agent; devices or other materials for preparing the polysaccharide preparation for administration; pharmaceutically acceptable carriers; and devices or other materials for administration to a subject. The instructions can include instructions for therapeutic application including suggested dosages and/or modes of administration, e.g., in a patient having a disorder, e.g., a disorder described herein. The kit can further contain at least one additional reagent, such as a diagnostic or therapeutic agent, e.g., a diagnostic or therapeutic agent as described herein, formulated as appropriate, in one or more separate pharmaceutical preparations.

### Uses

The polysaccharide preparations can be used to treat a subject. As used herein, a subject is a mammal, e.g., a non-human experimental mammal, a veterinary mammal, or a human. Non-human mammals include a primate, cow, horse, pig, sheep, goat, dog, cat, or rodent.

The preparations provided herein can be used, for example, to treat or prevent a metastatic disorder (e.g., a cancer, e.g., a carcinoma or other solid or hematological cancer). As used herein, the term "cancer" is meant to include all types of cancerous growths or oncogenic processes, metastatic tissues or malignantly transformed cells, tissues, or organs, irrespective of histopathologic type or stage of invasiveness. Methods and compositions disclosed herein are particularly useful for treating, or reducing the size, numbers, or rate of growth of, metastatic lesions associated with cancer.

Examples of cancers include, but are not limited to, solid tumors, soft tissue tumors, hematopoietic tumors and metastatic lesions. Examples of solid tumors include malignancies, e.g., sarcomas, adenocarcinomas, and carcinomas, of the various organ systems, such as those affecting head and neck (including pharynx), thyroid, lung (small cell or non small cell lung carcinoma), breast, lymphoid, gastrointestinal (e.g., oral, esophageal, stomach, liver, pancreas, small intestine, colon and rectum, anal canal), genitals and genitourinary tract (e.g., renal, urothelial, bladder, ovarian, uterine, cervical, endometrial, prostate, testicular), CNS (e.g., neural or glial cells, e.g., neorublastoma or glioma), skin (e.g., melanoma). Examples of hematopoietic cancers that can be treated include multiple myeloma, lymphomas and leukemias and myelodysplasia. Methods and compositions disclosed herein are particularly useful for treating, e.g., reducing or delaying, metastatic lesions associated with the aforementioned cancers. In some embodiments, the patient will have undergone one or more of surgical removal of a tissue, chemotherapy, or other anti-cancer therapy and the primary or sole target will be metastatic lesions, e.g., metastases in the bone or lymph nodes or lung or liver or peritoneal cavity or the CNS or other organs.

The methods of the disclosure can further include the step of monitoring the subject, e.g., for a change (e.g., an increase or decrease) in one or more of: tumor size; levels of a cancer marker, for a patient with cancer; the size or rate of appearance of new lesions, e.g., in a scan; the appearance of new disease-related symptoms; the size of soft tissue mass, e.g., a decrease or stabilization; quality of life, e.g., amount of disease associated pain, e.g., bone pain; or any other parameter related to clinical outcome. The subject can be monitored in one or more of the following periods: prior to beginning of treatment; during the treatment; or after one or more elements of the treatment have been administered. Monitoring can be used to evaluate the need for further treatment with the same preparation or for additional treatment with additional agents. Generally, a decrease in one or more of the parameters described above is indicative of the improved condition of the subject.

The preparations described herein can be administered to a subject in single or multiple doses to treat or prevent a metastatic or cancerous disorder, e.g., a cancerous disorder described herein.

The preparations described herein can also be used to treat inflammatory, autoimmune, fibrotic, fibroproliferative, atopic, or angiogenic disorders. Examples of inflammatory disorders include but are not limited to chronic obstructive pulmonary disease, asthma, rheumatoid arthritis, inflammatory bowel disease (including Crohns disease and ulcerative colitis), multiple sclerosis, psoriasis, ischemia-reperfusion injuries, septic shock, age-related macular degeneration, atherosclerosis, Alzheimer's disease, Parkinson's disease, cardiovascular disease, vasculitis, type I and II diabetes, metabolic syndrome, diabetic retinopathy, restenosis. Examples of autoimmune diseases include but are not limited to asthma, rheumatoid arthritis, inflammatory bowel disease, multiple sclerosis, psoriasis, type I diabetes, systemic lupus erythematosus (SLE), Sjögren's syndrome, Hashimoto's thyroiditis, Graves' disease, Guillain-Barré syndrome, autoimmune hepatitis, Myasthenia gravis. Examples of fibrotic diseases include but are not limited to scleroderma, chronic obstructive pulmonary disease, diabetic nephropathy, sarcoidosis, idiopathic pulmonary fibrosis, cirrhosis, cystic fibrosis, neurofibromatosis, endometriosis, post-operative fibroids, restenosis. Examples of atopic disease include but are not limited to atopic dermatitis, atopic asthma, and allergic rhinitis.

Examples of fibroproliferative disorders include systemic and local scleroderma, keloids and hypertrophic scars, atherosclerosis, restenosis, fibrosarcoma, neurofibromatosis, and rheumatoid arthritis. Examples of scarring associated with trauma include scarring due to surgery, chemotherapeutic-induced fibrosis, radiation-induced fibrosis, scarring associated with injury or burns.

In one embodiment, the polysaccharide preparations are used for inhibiting angiogenesis, e.g., to treat angiogenic disorders. Angiogenesis as used herein is the inappropriate formation of new blood vessels. Angiogenic disorders include, but are not limited to, tumors, neovascular disorders of the eye, endometriosis, macular degeneration, osteoporosis, psoriasis, arthritis, cancer and cardiovascular disorders. It is understood that some disorders will fall within more than one category of disease described herein.

The preparations described herein can also be used to treat or prevent infectious disorders such as, e.g., malaria.

### Combination Therapy

The methods and compositions of the disclosure can be used in combination with other therapeutic modalities. Administered "in combination", as used herein, means that two (or more) different treatments are delivered to the subject during the course of the subject's affliction with the disorder, such that the effects of the treatments on the patient overlap at a point in time. In some embodiments, the delivery of one treatment is still occurring when the delivery of the second begins, so that there is overlap in terms of administration. This is sometimes referred to herein as "simultaneous" or "concurrent delivery." In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

In one embodiment, the methods of the disclosure include administering to the subject a preparation described herein, in combination with one or more additional therapies, e.g., surgery, radiation therapy, or administration of another therapeutic preparation. In one embodiment, the additional therapy may include chemotherapy, e.g., a cytotoxic agent. In one embodiment the additional therapy may include a targeted therapy, e.g. a tyrosine kinase inhibitor, a proteasome inhibitor, a protease inhibitor. In one embodiment, the additional therapy may include an anti-inflammatory, anti-angiogenic, anti-fibrotic, or anti-proliferative compound, e.g., a steroid, a biologic immunomodulator, a monoclonal antibody, an antibody fragment, an aptamer, an siRNA, an antisense molecule, a fusion protein, a cytokine, a cytokine receptor, a bronchodialator, a statin, an anti-inflammatory agent (e.g. methotrexate), an NSAID. In another embodiment, the additional therapy could include combining therapeutics of different classes. The polysaccharide preparation and the additional therapy can be administered simultaneously or sequentially.

Exemplary cytotoxic agents that can be administered in combination with the polysaccharide preparation include antimicrotubule agents, topoisomerase inhibitors, antimetabolites, protein synthesis and degradation inhibitors, mitotic inhibitors, alkylating agents, platinating agents, inhibitors of nucleic acid synthesis, histone deacetylase and DNA methyltransferase inhibitors, nitrogen mustards, nitrosoureas, ethylenimines, alkyl sulfonates, triazenes, folate analogs, nucleoside analogs, ribnucleotide reductase inhibitors, vinca alkaloids, taxanes, epothilones, intercalating agents, agents capable of interfering with a signal transduction pathway, agents that promote apoptosis and radiation, antibody conjugates that bind surface proteins to deliver a toxic agent. In one embodiment, the cytotoxic agent that can be administered with a preparation described herein is platinum, cyclophosphamide, dacarbazine" methotrexate, fluorouracil, gemcitabine, capecitabine, hydroxyurea, topotecan, irinotecan, azacytidine, vorinostat, ixabepilone, bortezomib, taxanes (paclitaxel, docetaxel), cytochalasin B, gramicidin D, ethidium bromide, emetine, mitomycin, etoposide, tenoposide, vincristine, vinblastine, vinorelbine, colchicin, anthracyclines (doxorubicin and epirubicin) daunorubicin, dihydroxy anthracin dione, mitoxantrone, mithramycin, actinomycin D, adriamycin, 1-dehydrotestosterone, glucocorticoids, procaine, tetracaine, lidocaine, propranolol, puromycin, ricin, and maytansinoids.

The combination therapy can also include a composition of the present disclosure coformulated with, and/or coadministered with, one or more additional therapeutic agents, e.g., one or more anti-cancer agents, cytotoxic or cytostatic agents, hormone treatment, small molecule inhibitors of receptor tyrosine kinases and other tyrosine kinases including HER-2, EGFR, VEGFR, BCR-ABL, c-KIT (such as Gefitinib, Erlotinib, Lapatinib, Sorafenib, Sunitinib, Imatinib, Dasatinib, Nilotinib) or mTOR (such as temsirolimus, everolimus, rapamycin), or cytokines or chemokines, vaccines, antibodies against cell membrane receptors pathways including EGF-EGFR, VEGF-VEGFR, CD19, CD20, CD3, CTLA-4 (such as Trastuzumab, Cetuximab, Panitumumab, Bevacizumab, Rituximab, Tositumomab) and/or other immunotherapies.

### Other Embodiments

This invention is further illustrated by the following examples that should not be construed as limiting.

### EXAMPLES

### Example 1: Preparation of a Polysaccharide Preparation

This example describes the production of a polysaccharide preparation described herein.

**Overview:** Glycol Split low molecular weight heparin alcohol (GS-LMWH-CH₂-OH) was generated from unfractionated heparin (UFH) by controlled nitrous acid depolymerization followed by oxidative glycol-splitting and subsequent reduction to an alcohol. In the first step, UFH was depolymerized to obtain depolymerized heparin (DPH-CHO) having an anhydromannose moiety at the reducing end of the polysaccharide. This was followed by Step II oxidative cleavage of the 2, 3-diols present in the depolymerized heparin with sodium periodate to generate ring opened glycol split residues along the heparin chain (GS-DPH-CHO). The Step III involved a reduction step, wherein the aldehydic moieties are converted to alcohols using sodium borohydride to generate Glycol Split low molecular weight heparin alcohol.

### Method Overview:

The following paragraphs describe the preparation and properties of a polysaccharide preparation described herein.

### 1. Depolymerization:

Unfractionated Heparin (10 g) was dissolved in 100 mL of de-ionized water equilibrated at room temperature. The pH of this solution was subsequently lowered to pH 3.1, following which sodium nitrite (0.03 M) was added. This reaction solution was allowed to stir for 3 hours following which the pH was neutralized prior to addition of sodium chloride (10 g). After complete dissolution of salt, methanol (200 mL) was added to this solution with constant stirring. The precipitate obtained was then aged at 6 °C for 2 hours. This precipitate was then filtered and dried to obtain DPH in 80-85% yield and possessing the following characteristics:
Mw: 5300-6100
Mw Distribution:
   (i) <3000 Daltons: 23-30%
   (ii) 3000-8000 Daltons: 50-55%
   (iii) >8000 Daltons : 15-22%
Anti-Xa Activity: 80-120 IU/mg
Anti-IIa Activity: 40-70 IU/mg

### 2. Periodate Oxidation

The aldehyde (5 g) obtained in Step I was dissolved in 50 mL water equilibrated at 5 °C. To this solution was added cooled NaIO₄ solution (0.1 M, 50 mL) and the reaction mixture was allowed to stir in the absence of light for 16 hours. On completion, the reaction was quenched by the addition of diethylene glycol (10 mL), following which the temperature was raised back to room temperature. Five grams of sodium chloride was then added to this solution, followed by addition of 150 mL methanol to precipitate the heparin. The precipitate was allowed to age at 6 °C for 2 hours before filtration and drying to yield a glycol-split polysaccharide (95-98% yield) with the following characteristics:
Mw: 5000-5800
Mw Distribution:
   (i) <3000 Daltons: 25-30%
   (ii) 3000-8000 Daltons: 55-60%
   (iii) >8000 Daltons: 15-20%

### 3. Reduction

The glycol split polysaccharide (4 g) obtained above in Step II was dissolved in 40 mL water maintained at 5 °C. To this solution was added sodium borohydride (0.4 g) and the reaction mixture subsequently stirred for 1 hour. After 1 hour, the reaction mixture was brought to room temperature, followed by the addition of sodium chloride (4 g). Following salt dissolution, methanol (80 mL) was added to this solution accompanied with constant stirring. The precipitate thus obtained was then allowed to age at 6 °C for 2 hours before filtration and drying to yield the desired product. A polysaccharide preparation with the following characteristics was thus obtained in 55-60% yield:
Mw: 5500-6200
   Mw Distribution:
      (i) <3000 Daltons: 17-23%
      (ii) 3000-8000 Daltons: 56-62%
      (iii) >8000 Daltons: 17-22%
   Anti-Xa Activity: 5-20 IU/mg
   Anti-IIa Activity: 1-10 IU/mg

### Example 2: Anti-metastatic Properties of Polysaccharide Preparations

This example shows that the polysaccharide preparations have anti-cancer and antimetastatic activity in multiple models of metastasis.

### Model A: Murine melanoma experimental metastasis (B16F10 iv) model

A polysaccharide preparation produced as described in Example 1 (herein referred to as "MONC402") showed anti-metastasis activity in a murine melanoma experimental metastasis model.

Female C57BL/6 mice (9-10 weeks old) were treated once with a single dose (10 mg/kg) of MONC402, dalteparin/Fragmin® (a LMWH which has been reported to decrease metastasis), or MONC 202 (negative control, N-desulfated polysaccharide) immediately before i.v. injection of 2x10⁵ B16F10 cells. Mice were sacrificed on day 21 and tumor burden was calculated as lung weight-normal lung weight. As shown in Figure 1, MONC402 significantly inhibited B16F10 colonization of the lung relative to a pooled (untreated) control.

### Model B: Colon cancer metastasis to the liver

MONC402 showed prophylactic anti-metastasis activity in an orthotopic liver metastasis model.

Liver metastasis was initiated by intraperitoneal injection of C170HM2 human colorectal tumor cells into male MF1 nude (nu/nu) athymic mice. 5FU/leucovorin was used as a positive control.

C170HM2 cells were maintained in vitro in RPMI culture medium (Sigma) containing 10% (v/v) heat inactivated fetal bovine serum and 2 mM L-glutamine at 37 °C in 5% CO₂ and humidified conditions. Cells from sub-confluent monolayers were harvested with 0.025% EDTA, washed in culture medium and re-suspended in sterile phosphate buffered saline, pH 7.4 (PBS) for in vivo administration. 1.5 × 10⁶ cells in a volume of 1 ml were injected intraperitoneally into 65 mice, and the mice were allocated into treatment groups as below.

| | |
|---|---|
| Group 1: n = 10 | Vehicle control |
| Group 2: n = 10 | 25 mg/kg 5FU/leucovorin i.v. cycled on days 1, 3, 5, 7 |
| Group 3: n = 10 | 5 mg/kg compound 1 (Dalteparin) s.c. once daily |
| Group 4: n = 10 | 5 mg/kg compound 2 (MONC402) s.c. once daily |
| Group 5: n = 10 | 15 mg/kg compound 2 s.c. once daily |
| Group 6: n = 10 | 30 mg/kg compound 2 s.c. once daily |
| Group 7: n = 5 | Untreated |

Treatment was initiated on day 1 following cell injection and continued until day 35 or until the clinical condition of the animal required termination. Groups 5 and 6 missed one dose on day 5. No adverse affects of the test compounds in mice bearing the tumors were observed.

The study was terminated on day 35, and the tumors in the liver were excised and weighed. The numbers of lung nodules are also counted. The mean liver tumor weights and cross-sectional area are summarized in Table 1.

**Table 1. C170HM2 model: summary of mean liver tumor weight and statistical analysis**

| Group | Treatment | Mean tumor weight | | | Mean tumor area | | |
|---|---|---|---|---|---|---|---|
| | | (g) | (% of vehicle) | One way ANOVA | (mm²) | (% of vehicle) | One way ANOVA |
| 1 | Vehicle | 0.097 | 100.00 | - | 34.18 | 100.00 | - |
| 2 | 5FU/Leu | 0.037 | 11.94 | p = 0.006 | 13.12 | 15.7 | p = 0.011 |
| 3 | 5 mg/kg Dalteparin | 0.018 | 18.56 | p = 0.017 | 8.09 | 23/67 | p = 0.031 |
| 4 | 5 mg/kg MONC402 | 0.057 | 58.76 | NS | 18.34 | 53.66 | NS |
| 5 | 15 mg/kg MONC402 | 0.010 | 10.31 | p = 0.007 | 6.95 | 20.33 | p = 0.016 |
| 6 | 30 mg/kg MONC402 | 0.003 | 3.09 | p = 0.004 | 0.96 | 2.80 | p = 0.004 |
| 7 | Untreated control | 0.31 | - | p = 0.035 | 83.58 | 244.53 | p = 0.084 |

| | | | | | | | |
|---|---|---|---|---|---|---|---|
| NS = not significant | | | | | | | |

MONC 402 had significant influence in reducing tumor take rate. Both 15 mg/kg and 30 mg/kg MONC402 significantly reduced the liver tumor size by 90% (p = 0.007) and 97% (p = 0.004) respectively and also were significantly more effective than 5FU/leucovorin (p = 0.041 and p = 0.011, respectively). Dalteparin (group 3) reduced liver tumor weight by approximately 81% (p = 0.017) when compared to the vehicle control group. Similarly, the cross-sectional area of the tumors also showed significant reduction with dalteparin (p = 0.027) and 15 and 30 mg/kg MONC402 (p = 0.016 and p = 0.004, respectively).

Mouse weights were monitored for the duration of the study. The mouse weights for each group remained within an acceptable range for all groups throughout the study.

### Model C: Breast cancer metastasis to the lung

MONC402 also showed anti-metastasis activity in a syngeneic orthotopic model of breast cancer metastasis (4T1).

Female BALB/c mice 8 weeks of age (WOA) were injected with 8x10⁴ 4T1 cell intra mammary fat pad. Daily treatment with saline or MONC402 with or without weekly treatment of cisplatin started on day 5. Primary tumors were removed on day 9 and weighed.

As shown in Table 2, cisplatin combined with MONC402 (10, 20, 30 mg/kg) showed a statistically significant decrease in lung metastasis compared to saline control group as determined by lung weight and tumor nodule counting (p < 0.05, One way ANOVA). Combination therapy groups (Cisplatin + MONC402 10/20/30 mg/kg) also had lower incidence of mammary tumor regrowth, thoracic cavity tumor metastasis, and weight loss (> 2 g) in the last 3 days before termination of the experiment. Combination therapy groups had higher incidence of transient weight loss (> 2 g) the week after surgery but recovered in one week.

**Table 2. 4T1 model: macroscopic tumor metastasis counts**

| **groups** | **# of mice** | **Lung tumor nodule # /animal** | **Average lung tumor size** | **Total tumor nodule** | **Average tumor size** | **Total tumor volume** |
|---|---|---|---|---|---|---|
| **Saline** | 15 | 6.0 ±4.7 | 1.4 | 90 | 2.0 | 122.10 |
| **Cisplatin** | 16 | 5.6±4.4 | 1.41 | 89 | 1.36 | 134.13 |
| **MONC402 30mg/kg** | 16 | 8.4±7.0 | 1.19 | 135 | 1.11 | 140.98 |
| **Cisplatin+MONC402 30mg/kg** | 16 | 3.1±3.7 | 0.88 | 49 | 0.85 | 28.78 |
| **Cisplatin+MONC402 20mg/kg** | 15 | 2.3±2.9 | 0.8 | 34 | 1.0 | 18.66 |
| **Cisplatin+MONC402 10mg/kg** | 16 | 2.3±2.5 | 1.41 | 37 | 1.41 | 57.39 |
| **untreated** | 7 | 12.9±14.0 | 0.98 | 90 | 1.3 | 65.06 |

In a second 4T1 experiment, female BALB/c mice 8 WOA were injected with 8x10⁴ 4T1 cells intra mammary fat pad. Continuous osmotic pump delivery of saline or MONC 402 with weekly treatment of saline or Cisplatin started on day 4. Primary tumors were removed on day 9. There were no significant differences between the groups in primary tumor weight. However, immunohistology analyses showed significant decrease in microvessel density in tumors from mice treated with the combination of Cisplatin and M-ONC 402. The experiment terminated on day 32 and different samples were taken. 6 mice were either found dead or were terminated early due to worsened overall condition.

4T1 lung metastases were determined by lung weight, lung tumor nodule quantification including nodule number, size and calculated tumor volume, as well as histological quantification. Results are shown in Figure 2. MONC 402 (20 mg/kg/day) monotherapy groups did not significantly inhibit 4T1 lung metastasis. Cisplatin (1.25mg/kg) monotherapy showed significant anti-tumor efficacy (p<0.05). The combination of Cisplatin (1.25mg/kg) with MONC 402 (20mg/kg/day) displayed efficacy in reducing lung metastasis (p<0.0005) and reducing microvessel density. Importantly, the combination therapy group also showed better anti-tumor efficacy when compared to the cisplatin monotherapy group determined by lung weight (p<0.02), tumor nodule number, lung tumor coverage by histology, and lung tumor microvessel density (p<0.05, t-test), demonstrating M-ONC 402 enhanced the anti-tumor efficacy of cisplatin.

### Model D: Human prostate carcinoma PC-3MModel: combination therapy

Male SCID/Beige mice 8 WOA were injected with 5x10⁵ PC-3M-luciferase prostate carcinoma cells intra prostate. Daily treatment with saline or MONC402 with or without weekly treatment of cisplatin started on day 3. Mice were monitored weekly with Xenogen imaging system. The experiment was terminated on day 32. Different organs were isolated and tumor metastasis was assessed by weight and Xenogen imaging.

The MONC402 (30 mg/kg) monotherapy inhibited PC-3M metastasis in the peritoneum. Cisplatin combined with MONC402 (30 mg/kg) decreased tumor growth compared to saline and MONC 402 monotherapy groups as determined by in vivo imaging. There was no significant difference between combination therapy (Cisplatin + MONC402 30 mg/kg) and Cisplatin monotherapy in primary tumor weight and metastasis under the specific experimental condition.

## Claims

1. A polysaccharide preparation having the following characteristics:
a) the polysaccharide preparation has anti-Xa activity and anti-IIa activity each less than 50 IU/mg;
b) the polysaccharide preparation includes polysaccharides that include Formula I:
[U_{w}-H_{x,y,z}]ₘ~[U_{G}H_{x,y,z}]ₙ (I)
wherein
each occurrence of U indicates a uronic acid residue and each occurrence of H indicates a hexosamine residue;
m and n are integers such that
m = 4-16, and
n = 1-4;
each of w, x, y, and z can, independently, be the same or different for each occurrence of [U_{w}-H_{x,y,z}], and each of x, y, and z can, independently, be the same or different for each occurrence of [U_{G}-H_{x,y,z}];
w = -2OS or -2OH;
x = NS or NAc;
y = -3OS or -3OH;
z = -6OS or -6OH; and 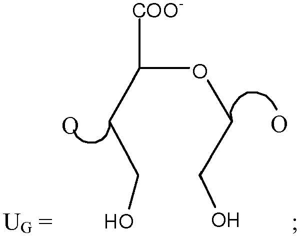 and wherein the symbol ~ indicates that the units marked m and n are distributed along the polysaccharide chain and are not necessarily in sequence;
c) the polysaccharide preparation has a molecular weight distribution such that 10-50% of the oligosaccharides of the preparation have a molecular weight < 3000 Da; 40-65% of the oligosaccharides have a molecular weight between 3000-8000 Da; and 5-30% of the oligosaccharides have a molecular weight > 8000 Da; and
d) the polysaccharide preparation has less than 25% glycol split uronic acid residues.

2. The polysaccharide preparation of claim 1, wherein the polysaccharide preparation has polysaccharide chains each having no more than 3 glycol split uronic acid residues (U_{G}).

3. The polysaccharide preparation of claim 1, wherein the polysaccharide preparation has polysaccharide chains having greater than 40% U_{2S}H_{NS,6S} disaccharide residues.

4. The polysaccharide preparation of claim 3, having a degree of desulfation less than 40%.

5. The polysaccharide preparation of any of the preceding claims, wherein one or more of the polysaccharide chains in the polysaccharide preparation have a reducing end which comprises a 2,5-anhydromannitol residue.

6. The polysaccharide preparation of claim 5, wherein about 50% of the polysaccharide chains in the polysaccharide preparation have reducing ends which comprise a 2,5-anhydromannitol residue.

7. The polysaccharide preparation of any of the preceding claims, wherein the polysaccharide preparation has a polydispersity of about 1.2 to 1.7, 1.3 to 1.7, 1.2 to 1.6 or 1.3 to 1.6.

8. The polysaccharide preparation of any of the preceding claims, wherein the polysaccharide preparation has one or more of a sodium content less than 30%, 25%, 20%, 15%, 10%; less than 20 ppm, 15 ppm, 10 ppm, 5 ppm iodine; less than 30%, 25%, 20%, 15%, 10% sulfur; and less than 50, 40, 30, 20, 15 ppm boron.

9. The polysaccharide preparation of any of the preceding claims, wherein n is 1-3.

10. The polysaccharide preparation of any of the preceding claims, wherein the polysaccharide preparation has a weight average chain molecular weight between 3,500 and 7,000 Da.

11. The polysaccharide preparation of any of the preceding claims, wherein the polysaccharide preparation has anti-Xa activity and anti-IIa activity each less than 20 IU/mg.

12. A pharmaceutical composition comprising a polysaccharide preparation of any of the preceding claims.

13. A polysaccharide preparation of any of claims 1-11 for use in a method of treating a metastatic disease; a VEGF-, FGF-, SDF-1α- and/or selectin-mediated disease; an inflammatory disease; an infectious disease; an autoimmune disease; fibrosis; or a disease involving angiogenesis in a subject.

14. The polysaccharide preparation for use of claim 13, wherein the polysaccharide preparation is administered chronically.

15. The polysaccharide preparation for use of claim 13, wherein the polysaccharide preparation is administered in combination with a second therapy, *e.g.* in combination with one or more additional therapeutic agents.

16. The polysaccharide preparation for use of claim 13, wherein the disease is a cancer.

17. The polysaccharide preparation for use of claim 16, wherein the cancer is a solid tumor, a soft tissue tumor, a hematopoietic tumor or a metastatic lesion.

18. The polysaccharide preparation for use of claim 16 or claim 17, wherein the cancer is a sarcoma, adenocarcinoma or a carcinoma of an organ system.

19. The polysaccharide preparation for use of claim 16 or claim 17, wherein the cancer is selected from head and neck cancer including pharyngeal cancer, thyroid cancer, lung cancer including small cell or non-small cell lung carcinoma, breast cancer, lymphoid cancer, gastrointestinal cancer including oral cancer, esophageal cancer, stomach cancer, liver cancer, pancreatic cancer, small intestinal cancer, colon cancer, rectal cancer and cancer of the anal canal, genital and genitourinary tract cancer including renal cell cancer, urothelial cancer, bladder cancer, ovarian cancer, uterine cancer, cervical cancer, endometrial cancer, prostate cancer and testicular cancer, cancer of the central nervous system including neural or glial cell cancer and skin cancer including melanoma.

20. The polysaccharide preparation for use of claim 19, wherein the cancer is pancreatic cancer.

21. The polysaccharide preparation for use of claim 19, wherein the cancer is colon cancer or rectal cancer.

22. The polysaccharide preparation for use of any of claims 16-21, wherein the polysaccharide preparation reduces or delays metastatic lesions associated with the cancer.

23. The polysaccharide preparation for use of any of claims 16-21, wherein the polysaccharide preparation is administered in combination with surgery, radiotherapy, a chemotherapeutic agent, an antibody or a tyrosine kinase inhibitor.

24. The polysaccharide preparation for use of claim 23, wherein the tyrosine kinase inhibitor inhibits a tyrosine kinase selected from HER-2, EGFR, VEGFR, BCR-ABL, c-KIT.

25. The polysaccharide preparation for use of claim 24, wherein the tyrosine kinase inhibitor is selected from Gefitinib, Erlotinib, Lapatinib, Sorafenib, Sunitinib, Imatinib, Dasatinib and Nilotinib.

26. The polysaccharide preparation for use of claim 23, wherein the antibody is against a cell membrane receptor pathway selected from EGF-EGFR, VEGF-VEGFR, CD19, CD20, CD3, and CTLA-4.

27. The polysaccharide preparation for use of claim 26, wherein the antibody is selected from Trastuzumab, Cetuximab, Panitumumab, Bevacizumab, Rituximab and Tositumomab.

28. The polysaccharide preparation for use of claim 23, wherein the chemotherapeutic agent is selected from an antimicrotubule agent, a topoisomerase inhibitor, an antimetabolite, a protein synthesis and degradation inhibitor, a mitotic inhibitor, an alkylating agent, a platinating agent, an inhibitors of nucleic acid synthesis, a histone deacetylase and DNA methyltransferase inhibitor, a nitrogen mustard, a nitrosourea, an ethylenimine, an alkyl sulfonate, a triazene, a folate analog, a nucleoside analog, a ribonucleotide reductase inhibitor, a vinca alkaloid, a taxane, an epothilone, an intercalating agent, an agent capable of interfering with a signal transduction pathway, an agent that promote apoptosis and an antibody conjugate that bind a surface protein to deliver a toxic agent.

29. The polysaccharide preparation for use of claim 23, wherein the chemotherapeutic agent is selected from a platinum, cyclophosphamide, dacarbazine, methotrexate, fluorouracil, gemcitabine, capecitabine, hydroxyurea, topotecan, irinotecan, azacytidine, vorinostat, ixabepilone, bortezomib, a taxane including paclitaxel and docetaxel, cytochalasin B, gramicidin D, ethidium bromide, emetine, mitomycin, etoposide, tenoposide, vincristine, vinblastine, vinorelbine, colchicin, an anthracycline including doxorubicin and epirubicin, daunorubicin, dihydroxy anthracin dione, mitoxantrone, mithramycin, actinomycin D, adriamycin, 1-dehydrotestosterone, a glucocorticoid, procaine, tetracaine, lidocaine, propranolol, puromycin, ricin, and a maytansinoid.

30. The polysaccharide preparation for use of claim 23, wherein the polysaccharide preparation is administered in combination with gemcitabine and/or a taxane (*e.g.* paclitaxel or docetaxel).

31. The polysaccharide preparation for use of any of claims 16-30, wherein the polysaccharide preparation is administered at dose of 5-50 mg/kg.

32. The polysaccharide preparation for use of any of claims 13-31, wherein the polysaccharide preparation is administered intravenously or subcutaneously.

33. The polysaccharide preparation for use of any of claims 13-32, wherein the polysaccharide preparation is administered daily.

34. A method of manufacturing a polysaccharide preparation of any of claims 1-11, the method comprising:
(1) depolymerizing an unfractionated heparin (UFH) with nitrous acid (HONO) to yield a depolymerized polysaccharide preparation;
(2) oxidizing the depolymerized polysaccharide preparation with periodate;
(3) reducing the oxidized polysaccharide preparation with sodium borohydride; and
(4) isolating the reduced polysaccharide preparation, to thereby make a polysaccharide preparation of any of claims 1-11;
wherein the step of depolymerizing comprises treating the UFH with about 0.02 to 0.04 M nitrous acid (HONO) at a pH of about 2 to 4 for about 1 to 5 hours at a temperature of about 10 to 30 °C.

35. The method of claim 34, wherein the step of oxidizing comprises treating the polysaccharide preparation with about 0.05 M to 0.2 M periodate for about 10 to 20 hours at a temperature of about 0 to 10 °C.

36. The method of claim 34, wherein the step of reducing comprises treating the oxidized polysaccharide preparation with about 0.5 to 2.0% (w/v) sodium borohydride for about 0.5 to 1.5 hours at a pH of about 6.0 to 7.0 and a temperature of about 0 to 10 °C.

37. A kit comprising a polysaccharide preparation of any of claims 1-11.

38. The kit of claim 37, further comprising one or more of: instructions for use; one or more additional therapeutic agents; devices or other materials for preparing the polysaccharide preparation for administration; pharmaceutically acceptable carriers; and devices or other materials for administration to a subject.

39. The kit of claim 38, wherein the instructions include a suggested dose and/or a mode of administration, for example, in a patient having a disorder.

40. The kit of claim 38, further comprising at least one additional reagent, such as a diagnostic or therapeutic agent.

41. The kit of any of claims 38-40, wherein the kit comprises one or more additional therapeutic agents.

42. The kit of claim 41, wherein the kit comprises one or more additional therapeutic agents as defined in any of claims 24-30.

43. The kit of claim 41, wherein the kit comprises gemcitabine and/or a taxane (*e.g.* paclitaxel or docetaxel).

## Patentansprüche

1. Polysaccharidzubereitung mit den folgenden Charakteristika:
a) die Polysaccharidzubereitung hat eine Anti-Xa-Aktivität und eine Anti-IIa-Aktivität von jeweils weniger als 50 IU/mg,
b) die Polysaccharidzusammensetzung umfasst Polysaccharide der Formel I:
[U_{w}-H_{x,y,z}]ₘ~[U_{G}-H_{x,y,z}]ₙ (I),
wobei
U jeweils für einen Uronsäurerest steht und H jeweils für einen Hexosaminrest steht,
m und n für ganze Zahlen stehen, so dass
m = 4-16 und
n = 1-4,
w, x, y und z jeweils unabhängig voneinander bei jedem Auftreten von [U_{w}-H_{x,y,z}] gleich oder verschieden sein können und x, y und z jeweils bei jedem Auftreten von [U_{G}-H_{x,y,z}] gleich oder verschieden sein können,
w = -2OS oder -2OH,
x = -NS oder -NAc,
y = -3OS oder -3OH,
z = -6OS oder -6OH und 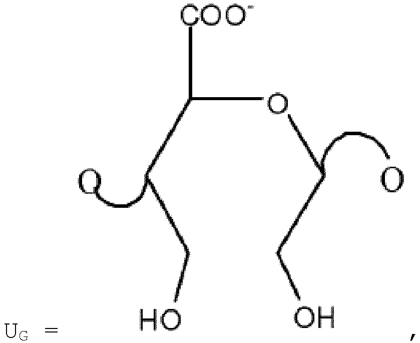 und wobei das Symbol ~ bedeutet, dass die mit m und n bezeichneten Einheiten entlang der Polysaccharidkette verteilt sind und nicht notwendigerweise geordnet sind,
c) die Molekulargewichtsverteilung der Polysaccharidzubereitung ist dergestalt, dass 10-50% der Oligosaccharide der Zubereitung ein Molekulargewicht von <3000 Da, 40-65% der Oligosaccharide ein Molekulargewicht zwischen 3000 und 8000 Da und 5-30% der Oligosaccharide ein Molekulargewicht > 8000 Da aufweisen, und
d) die Polysaccharidzubereitung hat weniger als 25% gykolgespaltene Uronsäurereste.

2. Polysaccharidzubereitung nach Anspruch 1, wobei die Polysaccharidzubereitung Polysaccharidketten mit jeweils nicht mehr als 3 glykolgespaltenen Uronsäureresten (U_{G}) aufweist.

3. Polysaccharidzubereitung nach Anspruch 1, wobei die Polysaccharidzubereitung Polysaccharidketten mit mehr als 40% U_{2S}H_{NS},_{6S}-Disaccharidresten aufweist.

4. Polysaccharidzubereitung nach Anspruch 3 mit einem Desulfatierungsgrad von weniger als 40%.

5. Polysaccharidzubereitung nach einem der vorhergehenden Ansprüche, wobei eine oder mehrere der Polysaccharidketten in der Polysaccharidzubereitung ein reduzierendes Ende aufweisen, welches einen 2,5-Anhydromannitolrest umfasst.

6. Polysaccharidzubereitung nach Anspruch 5, wobei etwa 50% der Polysaccharidketten in der Polysaccharidzubereitung reduzierende Enden aufweisen, die einen 2,5-Anhydromannitolrest umfassen.

7. Polysaccharidzubereitung nach einem der vorhergehenden Ansprüche, wobei die Polysaccharidzubereitung eine Polydispersität von etwa 1,2 bis 1,7, 1,3 bis 1,7, 1,2 bis 1,6 oder 1,3 bis 1,6 aufweist.

8. Polysaccharidzubereitung nach einem der vorhergehenden Ansprüche, wobei die Polysaccharidzubereitung einen oder mehrere der Folgenden aufweist: einen Natriumgehalt von weniger als 30%, 25%, 20%, 15%, 10%; weniger als 20 ppm, 15 ppm, 10 ppm, 5 ppm Iod; weniger als 30%, 25%, 20%, 15%, 10% Schwefel; und weniger als 50, 40, 30, 20, 15 ppm Bor.

9. Polysaccharidzubereitung nach einem der vorhergehenden Ansprüche, wobei n für 1-3 steht.

10. Polysaccharidzubereitung nach einem der vorhergehenden Ansprüche, wobei die Polysaccharidzubereitung ein gewichtsmittleres Kettenmolekulargewicht zwischen 3500 und 7000 Da aufweist.

11. Polysaccharidzubereitung nach einem der vorhergehenden Ansprüche, wobei die Polysaccharidzubereitung Anti-Xa-Aktivität und Anti-IIa-Aktivität von jeweils weniger als 20 IU/mg aufweist.

12. Pharmazeutische Zusammensetzung, umfassend eine Polysaccharidzubereitung nach einem der vorhergehenden Ansprüche.

13. Polysaccharidzubereitung nach einem der Ansprüche 1-11 zur Verwendung in einem Verfahren zur Behandlung einer metastatischen Krankheit, einer durch VEGF, FGF, SDF-1α und/oder Selectin vermittelten Krankheit, einer entzündlichen Krankheit, einer Infektionskrankheit, einer Autoimmunkrankheit, Fibrose oder einer Krankheit mit Angiogenese bei einem Subjekt.

14. Polysaccharidzubereitung zur Verwendung nach Anspruch 13, wobei die Polysaccharidzubereitung chronisch verabreicht wird.

15. Polysaccharidzubereitung zur Verwendung nach Anspruch 13, wobei die Polysaccharidzubereitung in Kombination mit einer zweiten Therapie, z.B. in Kombination mit einem oder mehreren zusätzlichen Therapeutika, verabreicht wird.

16. Polysaccharidzubereitung zur Verwendung nach Anspruch 13, wobei es sich bei der Krankheit um Krebs handelt.

17. Polysaccharidzubereitung zur Verwendung nach Anspruch 16, wobei es sich bei der Krebserkrankung um einen festen Tumor, einen Weichteiltumor, einen hämatopoietischen Tumor oder eine metastatische Läsion handelt.

18. Polysaccharidzubereitung zur Verwendung nach Anspruch 16 oder 17, wobei es sich bei der Krebserkrankung um ein Sarkom, ein Adenokarzinom oder Karzinom eines Organsystems handelt.

19. Polysaccharidzubereitung zur Verwendung nach Anspruch 16 oder 17, wobei die Krebserkrankung ausgewählt ist aus Kopf- und Halskrebs einschließlich Rachenkrebs, Schilddrüsenkrebs, Lungenkrebs einschließlich kleinzelligem oder nichtkleinzelligem Lungenkarzinom, Brustkrebs, lymphoidem Krebs, gastrointestinalem Krebs einschließlich Mundkrebs, Speiseröhrenkrebs, Magenkrebs, Leberkrebs, Bauchspeicheldrüsenkrebs, Dünndarmkrebs, Kolonkrebs, Rektalkrebs und Krebs des Analkanals, Krebs des Genital- und Urogenitaltrakts einschließlich Nierenzellkrebs, Urothelkrebs, Blasenkrebs, Eierstockkrebs, Uteruskrebs, Gebärmutterhalskrebs, Endometriumkrebs, Prostatakrebs und Hodenkrebs, Krebs des zentralen Nervensystems einschließlich Nervenzell- oder Gliazellkrebs und Hautkrebs einschließlich Melanom.

20. Polysaccharidzubereitung zur Verwendung nach Anspruch 19, wobei es sich bei der Krebserkrankung um Bauchspeicheldrüsenkrebs handelt.

21. Polysaccharidzubereitung zur Verwendung nach Anspruch 19, wobei es sich bei der Krebserkrankung um Kolonkrebs oder Rektalkrebs handelt.

22. Polysaccharidzubereitung zur Verwendung nach einem der Ansprüche 16-21, wobei die Polysaccharidzubereitung mit der Krebserkrankung assoziierte metastatische Läsionen reduziert oder verzögert.

23. Polysaccharidzubereitung zur Verwendung nach einem der Ansprüche 16-21, wobei die Polysaccharidzubereitung in Kombination mit einem operativen Eingriff, einer Radiotherapie, einem Chemotherapeutikum, einem Antikörper oder einem Tyrosinkinase-Inhibitor verabreicht wird.

24. Polysaccharidzubereitung zur Verwendung nach Anspruch 23, wobei der Tyrosinkinase-Inhibitor eine Tyrosinkinase ausgewählt aus HER-2, EGFR, VEGFR, BCR-ABL, c-KIT inhibiert.

25. Polysaccharidzubereitung zur Verwendung nach Anspruch 24, wobei der Tyrosinkinase-Inhibitor ausgewählt ist aus Gefitinib, Erlotinib, Lapatinib, Sorafenib, Sunitinib, Imatinib, Dasatinib und Nilotinib.

26. Polysaccharidzubereitung zur Verwendung nach Anspruch 23, wobei der Antikörper gegen einen Zellmembranrezeptorpfad ausgewählt aus EGF-EGFR, VEGF-VEGFR, CD19, CD20, CD3 und CTLA-4 gerichtet ist.

27. Polysaccharidzubereitung zur Verwendung nach Anspruch 26, wobei der Antikörper ausgewählt ist aus Trastuzumab, Cetuximab, Panitumumab, Bevacizumab, Rituximab und Tositumomab.

28. Polysaccharidzubereitung zur Verwendung nach Anspruch 23, wobei das Chemotherapeutikum ausgewählt ist aus einem Antimikrotubulusmittel, einem Topoisomerase-Inhibitor, einem Antimetaboliten, einem Proteinsynthese- und -abbau-Inhibitor, einem Mitose-Inhibitor, einem Alkylierungsmittel, einem Platinierungsmittel, einem Inhibitor der Nukleinsäuresynthese, einem Histondeacetylase- und DNA-Methyltransferase-Inhibitor, einem Stickstofflost, einem Nitrosoharnstoff, einem Ethylenimin, einem Alkylsulfonat, einem Triazen, einem Folatanalogon, einem Nukleosidanalogon, einem Ribonukleotidreduktase-Inhibitor, einem Vincaalkaloid, einem Taxan, einem Epothilon, einem Intercalierungsmittel, einem zur Störung eines Signalübertragungspfades fähigen Mittel, einem apoptosefördernden Mittel und einem zur Zurverfügungstellung eines toxischen Mittels an ein Oberflächenprotein bindenden Antikörperkonjugat.

29. Polysaccharidzubereitung zur Verwendung nach Anspruch 23, wobei das Chemotherapeutikum ausgewählt ist aus einem Platin, Cyclophosphamid, Dacarbazin, Methotrexat, Fluoruracil, Gemcitabin, Capecitabin, Hydroxyharnstoff, Topotecan, Irinotecan, Azacytidin, Vorinostat, Ixabepilon, Bortezomib, einem Taxan einschließlich Paclitaxel und Docetaxel, Cytochalasin B, Gramicidin D, Ethidiumbromid, Emetin, Mitomycin, Etoposid, Tenoposid, Vincristin, Vinblastin, Vinorelbin, Colchicin, einem Anthracyclin einschließlich Doxorubicin und Epirubicin, Daunorubicin, Dihdyroxyanthracindion, Mitoxantron, Mithramycin, Actinomycin D, Adriamycin, 1-Dehydrotestosteron, einem Glucocorticoid, Procain, Tetracain, Lidocain, Propranolol, Puromycin, Ricin und einem Maytansinoid.

30. Polysaccharidzubereitung zur Verwendung nach Anspruch 23, wobei die Polysaccharidzubereitung in Kombination mit Gemcitabin und/oder einem Taxan (z.B. Paclitaxel oder Docetaxel) verabreicht wird.

31. Polysaccharidzubereitung zur Verwendung nach einem der Ansprüche 16-30, wobei die Polysaccharidzubereitung in einer Dosis von 5-50 mg/kg verabreicht wird.

32. Polysaccharidzubereitung zur Verwendung nach einem der Ansprüche 13-31, wobei die Polysaccharidzubereitung intravenös oder subkutan verabreicht wird.

33. Polysaccharidzubereitung zur Verwendung nach einem der Ansprüche 13-32, wobei die Polysaccharidzubereitung täglich verabreicht wird.

34. Verfahren zur Herstellung einer Polysaccharidzubereitung nach einem der Ansprüche 1-11, wobei das Verfahren Folgendes umfasst:
(1) die Depolymerisierung eines nichtfraktionierten Heparins (Unfractionated Heparin, UFH) mit salpetriger Säure (HONO) unter Erhalt einer depolymerisierten Polysaccharidzubereitung,
(2) das Oxidieren der depolymerisierten Polysaccharidzubereitung mit Periodat,
(3) das Reduzieren der oxidierten Polysaccharidzubereitung mit Natriumborhydrid und
(4) das Isolieren der reduzierten Polysaccharidzubereitung unter Erhalt einer Polysaccharidzubereitung nach einem der Ansprüche 1-11,
wobei der Depolymerisierungsschritt die Behandlung des UFH mit etwa 0,02 bis 0,04 M salpetriger Säure (HONO) bei einem pH-Wert von etwa 2 bis 4 über etwa 1 bis 5 Stunden bei einer Temperatur von etwa 10 bis 30°C umfasst.

35. Verfahren nach Anspruch 34, wobei der Oxidationsschritt die Behandlung der Polysaccharidzubereitung mit etwa 0,05 M bis 0,2 M Periodat über 10 bis 20 Stunden bei einer Temperatur von etwa 0 bis 10°C umfasst.

36. Verfahren nach Anspruch 34, wobei der Reduktionsschritt die Behandlung der oxidierten Polysaccharidzubereitung mit etwa 0,5 bis 2,0% (w/v) Natriumborhydrid über etwa 0,5 bis 1,5 Stunden bei einem pH-Wert von etwa 6,0 bis 7,0 und einer Temperatur von etwa 0 bis 10°C umfasst.

37. Kit, umfassend eine Polysaccharidzubereitung nach einem der Ansprüche 1-11.

38. Kit nach Anspruch 37, weiterhin umfassend eines oder mehrere der Folgenden: Gebrauchsanweisung, ein oder mehrere zusätzliche Therapeutika, Geräte oder andere Materialien zur Vorbereitung der Polysaccharidzubereitung für die Verabreichung, pharmazeutisch unbedenkliche Träger, und Geräte oder andere Materialien für die Verabreichung an ein Subjekt.

39. Kit nach Anspruch 38, wobei die Anweisungen eine vorgeschlagene Dosis und/oder eine Verabreichungsweise, zum Beispiel bei einem Patienten mit einer Erkrankung, umfassen.

40. Kit nach Anspruch 38, welches weiterhin mindestens ein zusätzliches Reagenz wie ein Diagnostikum oder ein Therapeutikum umfasst.

41. Kit nach einem der Ansprüche 38-40, wobei das Kit ein oder mehrere zusätzliche Therapeutika umfasst.

42. Kit nach Anspruch 41, wobei das Kit ein oder mehrere wie in einem der Ansprüche 24-30 definierte zusätzliche Therapeutika umfasst.

43. Kit nach Anspruch 41, wobei das Kit Gemcitabin und/oder ein Taxan (z.B. Paclitaxel oder Docetaxel) umfasst.

## Revendications

1. Préparation de polysaccharides présentant les caractéristiques suivantes :
a) la préparation de polysaccharides présente une activité anti-Xa et une activité anti-IIa, chacune à moins de 50 UI/mg ;
b) la préparation de polysaccharides inclut des polysaccharides incluant la Formule I :
**[U_{w}-H_{x,y,z}]m~[U_{G}-H_{x,y,z}]ₙ** **(I)**
où
chacune des occurrences de U indique un résidu d'acide uronique, et chaque occurrence de H indique un résidu d'hexosamine ;
m et n sont des entiers tels que
m = 4-16, et n = 1-4 ;
chacun des nombres w, x, y et z peut être indépendamment identique ou différent pour chaque occurrence de [U_{w}-H_{x,y,z}], et chacun des nombres x, y et
z peut être indépendamment identique ou différent pour chaque occurrence de [U_{G}-H_{x,y,z}] ;
w = -2OS ou -2OH ;
x = -NS ou -NAc ;
y = -3OS ou -3OH ;
z = -6OS ou -6OH ;
et 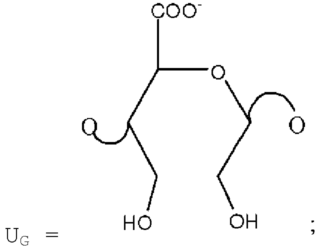 et où le symbole ~ indique que les unités marquées m et n sont distribués le long de la chaîne du polysaccharide et ne constituent pas nécessairement une séquence ;
c) la préparation de polysaccharides présente une distribution de masses moléculaires de sorte que 10 à 50 % des oligosaccharides de la préparation présentent une masse moléculaire < 3000 Da ; 40 à 65 % des oligosaccharides présentent une masse moléculaire comprise entre 3000 et 8000 Da ; et 5 à 30 % des oligosaccharides présentent une masse moléculaire > 8000 Da ; et
d) la préparation de polysaccharides présente moins de 25 % de résidus d'acide uronique divisés par glycol.

2. Préparation de polysaccharides selon la revendication 1, où la préparation de polysaccharides présente des chaînes de polysaccharides, chacune comportant moins de 3 résidus d'acide uronique divisés par glycol (U_{G}).

3. Préparation de polysaccharides selon la revendication 1, où la préparation de polysaccharides présente des chaînes de polysaccharides comportant plus de 40 % de résidus disaccharides U_{2S}H_{NS,6S}.

4. Préparation de polysaccharides selon la revendication 3, présentant un degré de désulfuration inférieur à 40 %.

5. Préparation de polysaccharides selon l'une quelconque des revendications précédentes, où une ou plusieurs des chaînes de polysaccharides de la préparation de polysaccharides présentent une extrémité réductrice qui comprend un résidu de 2,5-anhydromannitol.

6. Préparation de polysaccharides selon la revendication 5, où environ 50 % des chaînes de polysaccharides de la préparation de polysaccharides présentent des extrémités réductrices comprenant un résidu de 2,5-anhydromannitol.

7. Préparation de polysaccharides selon l'une quelconque des revendications précédentes, où la préparation de polysaccharides présente une polydispersité comprise entre environ 1,2 et 1,7, 1,3 et 1,7, 1,2 et 1,6 ou 1,3 et 1,6.

8. Préparation de polysaccharides selon l'une quelconque des revendications précédentes, où la préparation de polysaccharides présente une ou plusieurs des caractéristiques choisies parmi une teneur en sodium inférieure à 30 %, 25 %, 20 %, 15 %, 10 % ; moins de 20 ppm, 15 ppm, 10 ppm, 5 ppm d'iode ; moins de 30 %, 25 %, 20 %, 15 %, 10 % de soufre ; et moins de 50, 40, 30, 20, 15 ppm de bore.

9. Préparation de polysaccharides selon l'une quelconque des revendications précédentes, où n est compris entre 1 et 3.

10. Préparation de polysaccharides selon l'une quelconque des revendications précédentes, où la préparation de polysaccharides présente une masse moléculaire de chaîne moyenne en poids entre 3500 et 7000 Da.

11. Préparation de polysaccharides selon l'une quelconque des revendications précédentes, où la préparation de polysaccharides présente une activité anti-Xa et une activité anti-IIa, chacune étant inférieure à 20 UI/mg.

12. Composition pharmaceutique comprenant une préparation de polysaccharides selon l'une quelconque des revendications précédentes.

13. Préparation de polysaccharides selon l'une quelconque des revendications 1 à 11 pour utilisation dans une méthode de traitement d'une maladie métastatique ; d'une maladie faisant intervenir VEGF, FGF, SDF-la et/ou la sélectine ; d'une maladie inflammatoire ; d'une maladie infectieuse ; d'une maladie auto-immune ; d'une fibrose ; ou d'une maladie impliquant une angiogenèse chez un sujet.

14. Préparation de polysaccharides pour utilisation selon la revendication 13, où la préparation de polysaccharides est administrée de façon chronique.

15. Préparation de polysaccharides pour utilisation selon la revendication 13, où la préparation de polysaccharides est administrée en combinaison avec un deuxième traitement, par exemple en combinaison avec un ou plusieurs agents thérapeutiques supplémentaires.

16. Préparation de polysaccharides pour utilisation selon la revendication 13, où la maladie est un cancer.

17. Préparation de polysaccharides pour utilisation selon la revendication 16, où le cancer est une tumeur solide, une tumeur des tissus mous, une tumeur hématopoïétique ou une lésion métastatique.

18. Préparation de polysaccharides pour utilisation selon la revendication 16 ou la revendication 17, où le cancer est un sarcome, un adénocarcinome ou un carcinome d'un système d'organes.

19. Préparation de polysaccharides pour utilisation selon la revendication 16 ou la revendication 17, où le cancer est choisi parmi le cancer de la tête et du cou y compris cancer du pharynx, le cancer de la thyroïde, le cancer du poumon y compris les carcinomes à petites cellules ou non à petites cellules, le cancer du sein, le cancer lymphoïde, le cancer gastro-intestinal y compris les cancers oraux, le cancer de l'oesophage, le cancer de l'estomac, le cancer du foie, le cancer du pancréas, le cancer de l'intestin grêle, le cancer du côlon, le cancer du rectum et le cancer du canal anal, le cancer de l'appareil génital et urogénital y compris cancer à cellules rénales, cancer urothélial, cancer de la vessie, cancer de l'ovaire, cancer de l'utérus, le cancer du col de l'utérus, cancer de l'endométrium, cancer de la prostate et cancer du testicule, le cancer du système nerveux central y compris cancer à cellules neurales ou gliales et le cancer de la peau y compris mélanomes.

20. Préparation de polysaccharides pour utilisation selon la revendication 19, où le cancer est le cancer du pancréas.

21. Préparation de polysaccharides pour utilisation selon la revendication 19, où le cancer est le cancer du côlon ou le cancer du rectum.

22. Préparation de polysaccharides pour utilisation selon l'une quelconque des revendications 16 à 21, où la préparation de polysaccharides réduit ou retarde les lésions métastatiques associées au cancer.

23. Préparation de polysaccharides pour utilisation selon l'une quelconque des revendications 16 à 21, où la préparation de polysaccharides et administrés en combinaison avec une chirurgie, une radiothérapie, un agent chimiothérapique, un anticorps ou un inhibiteur de tyrosine kinase.

24. Préparation de polysaccharides pour utilisation selon la revendication 23, où l'inhibiteur de tyrosine kinase inhibe une tyrosine kinase choisie parmi HER-2, EGFR, VEGFR, BCR-ABL, c-KIT.

25. Préparation de polysaccharides pour utilisation selon la revendication 24, où l'inhibiteur de tyrosine kinase est choisi parmi Géfitinib, Erlotinib, Lapatinib, Sorafénib, Sunitinib, Imatinib, Dasatinib et Nilotinib.

26. Préparation de polysaccharides pour utilisation selon la revendication 23, où l'anticorps est dirigé contre une voie des récepteurs membranaires cellulaires choisie parmi EGF-EGFR, VEGF-VEGFR, CD19, CD20, CD3 et CTLA-4.

27. Préparation de polysaccharides pour utilisation selon la revendication 26, où l'anticorps est choisi parmi les suivants : Trastuzumab, Cétuximab, Panitumumab, Bévacizumab, Rituximab et Tositumomab.

28. Préparation de polysaccharides pour utilisation selon la revendication 23, où l'agent chimiothérapique est choisi parmi un agent antimicrotubulaire, un inhibiteur de topoisomérase, un antimétabolite, un inhibiteur de synthèse et de dégradation des protéines, un inhibiteur de mitose, un agent d'alkylation, un agent de platination, un inhibiteur de synthèse d'acide nucléique, une histone désacétylase et un inhibiteur d'ADN de méthyltransférase, une moutarde azotée, une nitrosourée, une éthylèneimine, un sulfonate d'alkyle, un triazène, un analogue de folate, un analogue de nucléoside, un inhibiteur de ribonucléotide réductase, un vinca-alkaloïde, un taxane, une épothilone, un agent d'intercalation, un agent capable d'interférer avec une voie de transduction du signal, un agent favorisant l'apoptose et un conjugué d'anticorps qui se lie à une protéine de surface pour libérer un agent toxique.

29. Préparation de polysaccharides pour utilisation selon la revendication 23, où l'agent chimiothérapique est choisi parmi les suivants : platine, cyclophosphamide, dacarbazine, méthotrexate, fluorouracile, gemcitabine, capécitabine, hydroxyurée, topotécane, irinotécane, azacytidine, vorinostat, ixabépilone, bortézomib, taxane y compris paclitaxel et docétaxel, cytochalasine B, gramicidine D, bromure d'éthidium, émétine, mitomycine, étoposide, ténoposide, vincristine, vinblastine, vinorelbine, colchicine, anthracycline y compris doxorubicine et épirubicine, daunorubicine, dihydroxyanthracine dione, mitoxantrone, mithramycine, actinomycine D, adriamycine, 1-déshydrotestostérone, glucocorticoïde, procaïne, tétracaïne, lidocaïne, propranolol, puromycine, ricine, et maytansinoïde.

30. Préparation de polysaccharides pour utilisation selon la revendication 23, où la préparation de polysaccharides est administrée en combinaison avec la gemcitabine et/ou un taxane (par exemple paclitaxel ou docétaxel).

31. Préparation de polysaccharides pour utilisation selon l'une quelconque des revendications 16 à 30, où la préparation de polysaccharides est administrée à une dose de 5 à 50 mg/kg.

32. Préparation de polysaccharides pour utilisation selon l'une quelconque des revendications 13 à 31, où la préparation de polysaccharides est administrée par voie intraveineuse ou sous-cutanée.

33. Préparation de polysaccharides pour utilisation selon l'une quelconque des revendications 13 à 32, où la préparation de polysaccharides est administrée quotidiennement.

34. Procédé de fabrication d'une préparation de polysaccharides selon l'une quelconque des revendications 1 à 11, le procédé comprenant :
(1) dépolymérisation d'une héparine non fractionnée (UFH) par de l'acide nitreux (HONO) pour obtenir une préparation de polysaccharides dépolymérisée ;
(2) oxydation de la préparation de polysaccharides dépolymérisée par du periodate ;
(3) réduction de la préparation de polysaccharides oxydée par du borohydrure de sodium ;
et
(4) isolement de la préparation de polysaccharides réduite, pour obtenir une préparation de polysaccharides selon l'une quelconque des revendications 1 à 11 ;
où l'étape de dépolymérisation comprend le traitement de l'UFH par environ 0,02 à 0,04M d'acide nitreux (HONO) à un pH d'environ 2 à 4 pendant environ 1 à 5 heures à une température d'environ 10 à 30 °C.

35. Procédé selon la revendication 34, où l'étape d'oxydation comprend le traitement de la préparation de polysaccharides par environ 0,05M à 0,2M de periodate pendant environ 10 à 20 heures à une température d'environ 0 à 10 °C.

36. Procédé selon la revendication 34, où l'étape de réduction comprend le traitement de la préparation de polysaccharides oxydée par environ 0,5 à 2,0 % en masse par volume de borohydrure de sodium pendant environ 0,5 à 1,5 heure à un pH d'environ 6,0 à 7,0 et une température d'environ 0 à 10 °C.

37. Kit comprenant une préparation de polysaccharides selon l'une quelconque des revendications 1 à 11.

38. Kit selon la revendication 37, comprenant en outre un ou plusieurs des éléments suivants :
instructions d'utilisation ; un ou plusieurs agents thérapeutiques supplémentaires ; dispositifs ou autres matériels d'élaboration de la préparation de polysaccharides pour administration ; vecteurs pharmaceutiquement acceptables ; et dispositifs ou autres matériels pour administration à un sujet.

39. Kit selon la revendication 38, où les instructions incluent une suggestion de dose et/ou de mode d'administration, par exemple chez un patient présentant un trouble.

40. Kit selon la revendication 38, comprenant en outre au moins un réactif supplémentaire, tel qu'un agent diagnostique ou thérapeutique.

41. Kit selon l'une quelconque des revendications 38 à 40, où le kit comprend un ou plusieurs agents thérapeutiques supplémentaires.

42. Kit selon la revendication 41, où le kit comprend un ou plusieurs agents thérapeutiques supplémentaires selon l'une quelconque des revendications 24 à 30.

43. Kit selon la revendication 41, où le kit comprend de la gemcitabine et/ou un taxane (par exemple paclitaxel ou docétaxel).
